# EUROPEAN PATENT SPECIFICATION

(11) **EP 0 828 749 B1**
(45) Date of publication and mention of the grant of the patent: **16.07.2003**
(21) Application number: 96917829.2
(22) Date of filing: 24.05.1996
(51) Int. Cl.: C07H 19/04, C07H 19/10, C07H 19/048, C07H 19/20, A61K 31/70

(54) **METHODS FOR THE SYNTHESIS OF ORGANOPHOSPHORUS DERIVATIVES**
VERFAHREN ZUR SYNTHESE VON PHOSPHORORGANISCHEN VERBINDUNGEN
PROCEDES DE SYNTHESE DE DERIVES ORGANOPHOSPHOREUX

(30) Priority: 26.05.1995 PL 30880395; 01.09.1995 PL 31024895; 26.02.1996 PL 31293496
(43) Date of publication of application: 18.03.1998
(60) Divisional application: 03001247.0
(73) Proprietor: GENTA INCORPORATED, San Diego, California 92121 (US); Polska Akademia Nauk, 90-363 Lodz (PL)
(72) Inventor: STEC, Wojciech, J., PL-95-054 Ksawerow (PL); WOZNIAK, Lucyna, PL-90-369 Lodz (PL); RILEY, Timothy, San Diego, CA 92129 (US)
(74) Representative: Hansen, Bernd, Dr. Dipl.-Chem.
(86) International application number: US9607639
(87) International publication number: WO96037503

(56) References cited:
- EP-A- 0 136 543
- L. A. WOZNIAK ET AL.: "New stereospecific Method of Synthesis of dinucleoside methasulphonates" THE JOURNAL OF ORGANIC CHEMISTRY, vol. 59, no. 20, - 7 October 1994 pages 5843-5846, XP002074478
- W. BRILL AND M. CARUTHERS: "Synthesis of P-Diastereomeric resolution of nucleoside 3'-o-(s-alkyl) and nucleoside 3'-o(s-aryl) methylphosphonothioates" TETRAHEDRON LETTERS, vol. 29, no. 11, - 1988 pages 1227-1230, XP002074479

## Description

### Technical Field

The present invention relates generally to methods of synthesis for organophosphorus mono - and di - nucleoside derivatives.

### Background of the Invention

It is well known that most of the bodily states in animals, including disease states, are effected by proteins. Such proteins, either acting directly or through their enzymatic functions contribute to many disease states in animals and man.

Classical therapeutics has generally focused upon interactions with such proteins in an effort to moderate their disease causing or disease potentiating functions. Recently, however, attempts have been made to moderate the actual production of such proteins by interactions with the molecules (*i.e.* intercellular RNA) that direct their synthesis. These interactions have involved the hybridization of complimentarity "antisense" oligonucleotides or certain analogs thereof to RNA. Hybridization refers to the sequence-specific hydrogen bonding of oligonucleotides or oligonucleotide analogs to RNA or DNA. When hybridization occurs biosynthesis of proteins can be interrupted. This interference with the production of proteins, has been hoped to effect therapeutic results with maximum effect and minimal side effects. Oligonucleotide analogs may also be utilized to moderate the production of proteins by a similar mechanism.

The pharmacological activity of antisense oligonucleotides and oligonucleotide analogs, like other therapeutics, depends on a number of factors that influence the effective concentration of these agents at specific intercellular targets. One important factor for oligonucleotides is the stability of the species in the presence of nucleases. It is unlikely that unmodified oligonucleotides will be useful therapeutic agents because they are rapidly degraded by nucleases. Modifications of oligonucleotides to render them resistant to nucleases therefore are greatly desired.

Modifications of oligonucleotides to enhance nuclease resistance have generally taken place on the phosphorus atom of the sugar-phosphate backbone. Phosphorothioates, methyl phosphonates, phophoramidates, and phosphorotriesters have been reported to confer various levels of nuclease resistance. However, phosphate-modified oligonucleotides of this type generally have suffered from inferior hybridization properties (Cohen, J.S., ed. *Oligonucleotides: Antisense Inhibitors of Gene Expression,* CRC Press, Inc. Boca Raton FL, 1989)

Another key factor is the ability of antisense compounds to traverse the plasma membrane of specific cells involved in the disease process. Cellular membranes consist of lipid-protein bilayers that are freely permeable to small, nonionic, lipophilic compounds yet inherently impermeable to most natural metabolites and therapeutic agents (Wilson, D.B. *Ann. Rev. Biochem.* 47:933, 1978) The biological and antiviral effects of natural and modified oligonucleotides in cultured mammalian cells have been well documented. Thus, it appears that these agents can penetrate membranes to reach their intercellular targets. Uptake of antisense compounds by a variety of mammalian cells including HL-60, Syrian Hamster fibroblast, U937, L929, CV-1 and ATH8 cells, have been studied using natural oligonucleotides and certain nuclease resistant analogs, such as alkyl triesters (Miller P.S. *et al., Biochem. 16*:1988, 1977); methylphosphonates (Marcus-Sekura, C.H. *et al., Nuc. Acids Res. 15*:5749, 1987; Miller P.S. *et al., Biochem. 16*:1988, 1977; and Loke S.K. *et al., Top. Microbial. Immunol. 141*:282, 1988).

Modified oligonucleotides and oligonucleotide analogs may be less readily internalized than their natural counterparts. As a result, the activity of many previously available antisense oligonucleotides have not been sufficient for practical therapeutic, research or diagnostic purposes. Two other deficiencies recognized by the prior art are that many of the previously designed oligonucleotide antisense therapeutics hybridize less efficiently to intercellular RNA and lack the defined chemical or enzyme-mediated event to terminate essential RNA function.

Modifications to enhance the effectiveness of the antisense oligonucleotides and overcome these problems have taken many forms. These modifications include base ring modifications, sugar moiety modifications, and sugar-phosphate backbone modifications. Prior sugar-phosphate backbone modifications, particularly on the phosphorus atom, have effected various levels of resistance to nucleases. However, while the ability of an antisense oligonucleotide to bind to specific DNA or RNA with fidelity is fundamental to antisense methodology, modified phosphorus oligonucleotides have generally suffered from inferior hybridization properties.

Replacement of the phosphorus atom has been an alternative approach in attempting to avoid the problems associated with modification on the prochiral phosphate moiety. Some modifications in which replacement of the phosphorus atom has been achieved are discussed by Matteucci, (*Tetrahedron Letters 31*:2385, 1990), wherein replacement of the phosphorus atom with a methylene group is limited by available methodology which does not provide for uniform insertion of the formacetal linkage throughout the backbone, and its instability, making it unsuitable for use; Cormier, *(Nuc. Acids Res. 16*:4583, 1988), wherein replacement of the phosphorus moiety with a diisopropylsilyl moiety is limited by methodology, solubility of the homopolymers and hybridization properties; Stirchak *(J. Org. Chem. 52*:4202, 1987), wherein replacement of the phosphorus linkage by short homopolymers containing carbamate or morpholino linkages is limited by methodology, the solubility of the resulting molecule, and hybridization properties; Mazur (*Tetrahedron 40*:3949, 1984), wherein replacement of the phosphorus linkage with a phosphonic linkage has not been developed beyond the synthesis of a homotrimer molecule; and Goodrich (*Bioconj. Chem. 1*:165, 1990) wherein ester linkages are enzymatically degraded by esterases and are therefore unsuitable to replace the phosphonate bond in antisense applications.

Another key factor are the sterochemical effects of that arise in oligomers having chiral centers. In general, an oligomer with a length of n nucleosides will constitute a mixture of 2ⁿ⁻¹ isomers in successive non-stereospecific chain synthesis.

It has been observed that Rp and Sp homochiral chains, whose absolute configuration at all internucleotide methanephosphonate phosphorus atoms is either Rp or Sp, and non-stereoregular chains show different physicochemical properties as well as different capabilities of forming adducts with oligonucleotides of complementary sequence. In addition, phosphorothioate analogs of nucleotides have shown substantial stereoselectivity differences between Oligo-Rp and Oligo-Sp oligonucleotides in resistance to nucleases activity (Potter, *Biochemistry,* 22:1369, 1983; Bryant *et al., Biochemistry, 18*:2825, 1979).

Lesnikowski (*Nucl. Acids Res.,* 18:2109, 1990 observed that diastereomeric pure octathymidine methanephosphonates, in which six out of seven methanephosphonate bonds have defined configuration at the phosphorus atom when complexed with a the matrix of pentadecadeoxyriboadenylic acid show substantial differences in melting temperatures. The Oligonucleotide compounds with predetermined configuration at the phosphorus atom, used in these studies, were prepared by the stereocontrolled process between the 5'-hydroxyl nucleoside group activated by means of the Grignard's reagent, and the diastereomerically pure nucleoside p-nitrophenylmethanephosphonate (Lesnikowski et al., *Nucl. Acids Res.,* 18:2109, 1990; Lesnikowski *et al., Nucleosides & Nucleotides,* 10:773, 1991; Lesnikowski, *Nucl. Acids Res.,* 16:11675, 1988). This method, however, requires long reaction time, and has been verified only in the case of the synthesis of tetramer homothymidine fragments and heteromeric hexamers.

Attempts to prepare diastereomerically pure oligomethylphosphonate compounds by reacting at low temperatures (-80°C) with methyldichlorophosphine and appropriate nucleosides protected at 5' or 3' positions, resulted in the formation of Rp isomers of relevant dinucleoside methylphosphonates at a maximum predominace of 8:1 (Loschner, *Tetrahedron Lett*., 30:5587, 1989; and Engels *et al., Nucleosides & Nucleotides*, 10:347, 1991).

Wozniak L. A., et al. (The Journal of Organic Chemistry), Vol. 59, No. 20, 1994, pp. 5843 bis 5846) describes a new stereospecific method of synthesis of (Sp)- and (Rp)-Dinucleoside-(3',5') methanephosphonates. Brill and Caruthers describe in "Tetrahedron Letters, Vol. 29, No. 11, pp. 1227-1230" the synthesis and P-diastereomeric resolution of nucleoside 3'-O(S-Aryl) methylphosphonothioates. In EP 0 136 543, a method for the preparation of alkyl-, aralkyl-, and arylphosphonites and phosphonates nucleosides is described.

However, longer stereoregular chains cannot be prepared by this method because intermediate nucleoside 3'-O-chloromethylphosphonites, formed during the condensation, have a labile configuration even at low temperatures.

The limitations of the available methods for modification and synthesis of the organophosphorus derivatives have led to a continuing and long felt need for other modifications which provide resistance to nucleases and satisfactory hybridization properties for antisense oligonucleotide diagnostics, therapeutics, and research.

### Summary of the Invention

According to the invention, methods are provided for the synthesis of organophosphorus derivative dinucleosides utilizing monomeric organophosphorus derivatives.

In one embodiment a method for the synthesis of a diastereomeric mixture of P-chiral dinucleosides of formulas (6a) and (6b) 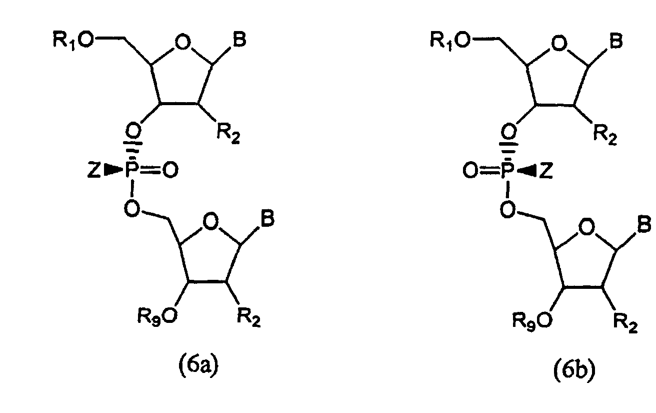 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (8a) and (8b) 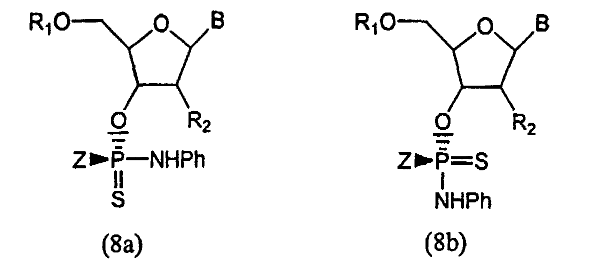 with sodium hydride or 1, 8-diazabicyclo[5.4.0]undec-7-ene and carbon dioxide to form transient nucleosides 3'-O-(Z-substituted)phosphonothioic acids;
(b) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (7a) and (7b) 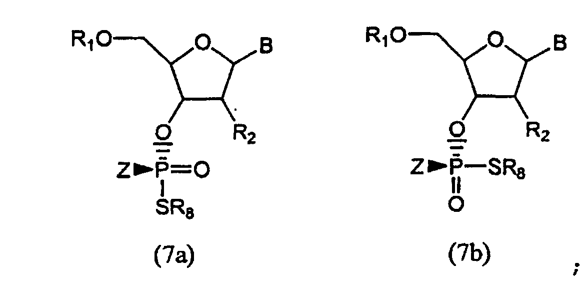 and
(c) reacting the alkylated nucleoside intermediates with a nucleoside of formula (5) 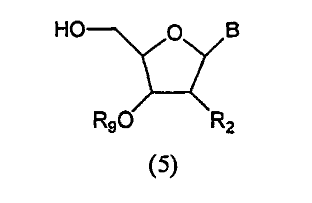 in presence of an activator and a lithium salt in an aprotic organic solvent to form the diastereomeric mixture of P-chiral dinucleosides of formulas (6a) and (6b).

In another embodiment a method for the synthesis of a diastereomeric mixture of P-chiral dinucleosides of formulas (6a) and (6b) 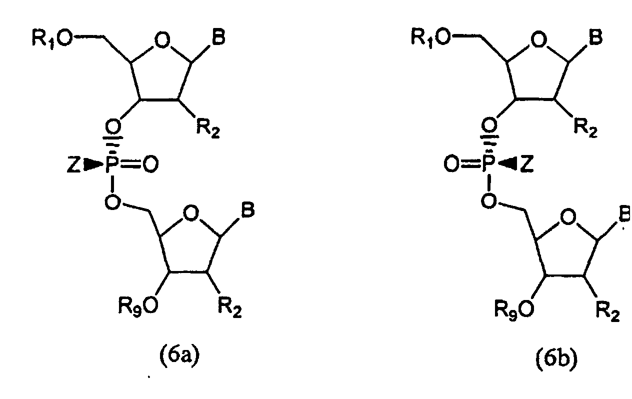 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (10a) and (10b) 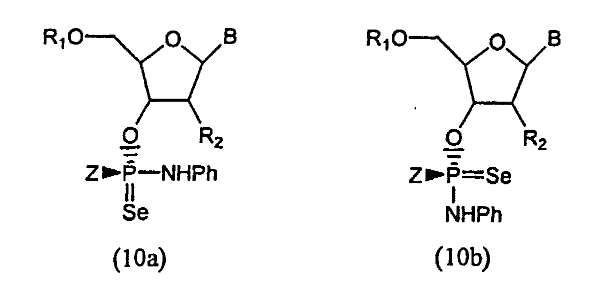 with sodium hydride or 1, 8-diazabicyclo[5.4.0]undec-7-ene and carbon dioxide to form transient nucleosides 3'-O-(Z-substituted)phosphonoselenoic acids;
(b) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (11a) and (11b) 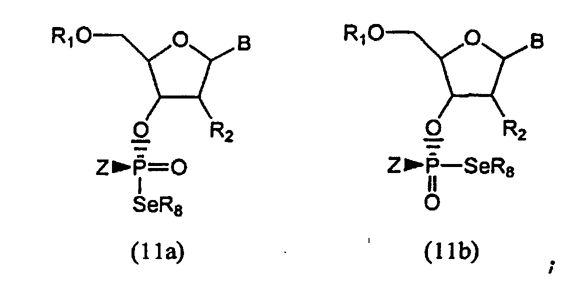 and
(c) reacting the alkylated nucleoside intermediates with a nucleoside of formula (5) 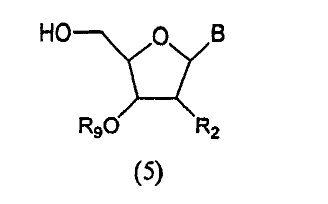 in presence of an activator and a lithium salt in an aprotic organic solvent to form the diastereomeric mixture of P-chiral dinucleosides of formulas (6a) and (6b).

In still another embodiment, a method for the synthesis of a diastereomeric mixture of P-chiral dinucleosides of formulas (6a) and (6b) 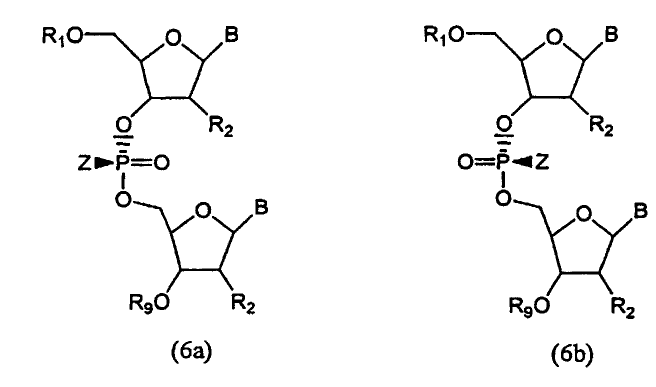 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (8a) and (8b) 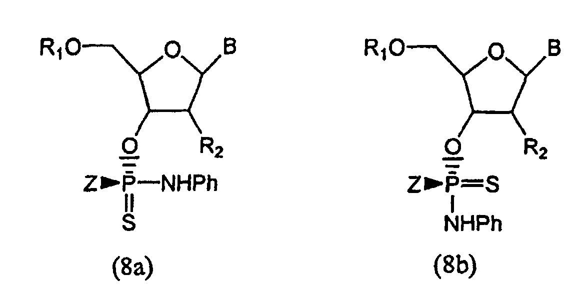 with potassium peroxymonosulfate or hydrogen peroxide to form nucleoside intermediates of formulas (9a) and (9b) 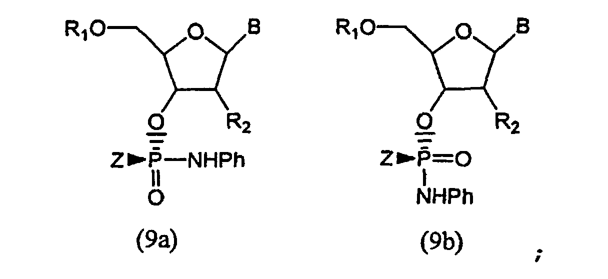
(b) reacting the nucleoside intermediates with sodium hydride or 1, 8-diazabicyclo[5.4.0]undec-7-ene and carbon disulfide to form transient nucleosides 3'-O-(Z-substituted)phosphonothioic acids;
(c) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (7a) and (7b) 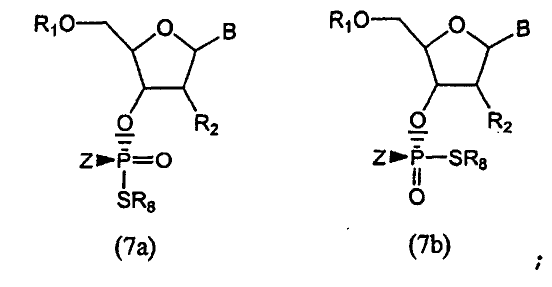 and
(d reacting the alkylated nucleoside intermediates with a nucleoside of formula (5) 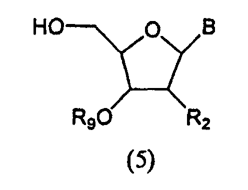 in presence of an activator and a lithium salt in an aprotic organic solvent to form the diastereomeric mixture of P-chiral dinucleosides of formulas (6a) and (6b).

In still another embodiment a method for the synthesis of a diastereomeric mixture of P-chiral dinucleosides of formulas (6a) and (6b) 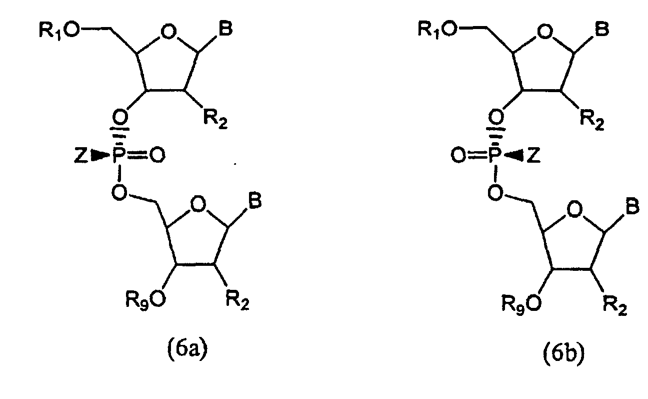 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formula (10a) and (10b) 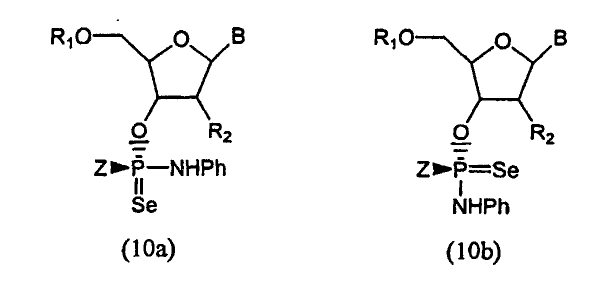 with potassium peroxymonosulfate or hydrogen peroxide to form nucleoside intermediates of formulas (9a) and (9b) 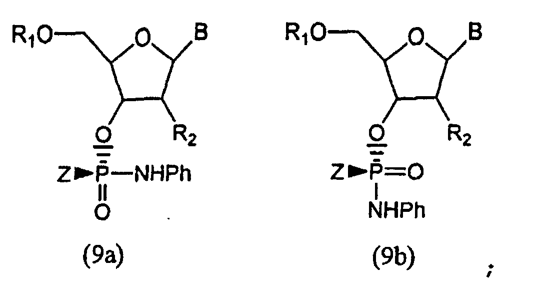
(b) reacting the nucleoside intermediates with sodium hydride or DBU and carbon diselenide to form transient nucleosides 3'-O-(Z-substituted)phosphonoselenoic acids;
(c) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (11a) and (11b) 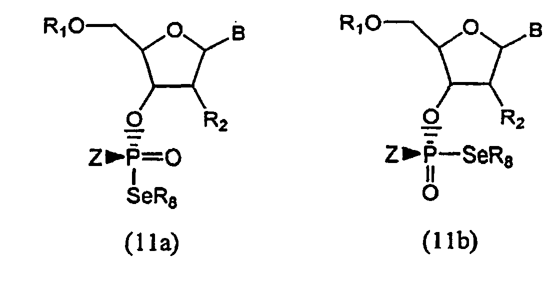 and
(d) reacting the alkylated nucleoside intermediates with a nucleoside of formula (5) 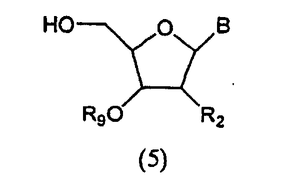 in presence of an activator and a lithium salt in an aprotic organic solvent to form the diastereomeric mixture of P-chiral dinucleosides of formulas (6a) and (6b).

In another aspect of this invention, methods for the synthesis of chirally pure organophosphorus derivative dinucleotides is provided a method for the synthesis of a chirally pure dinucleoside of formula (6a) 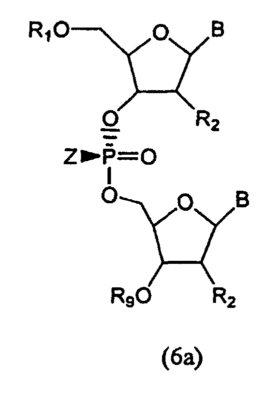 wherein
R₁ is a protecting group;
R₂ is H, OH, or alkoxy moiety of 1 to 10 carbon atoms;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8a) 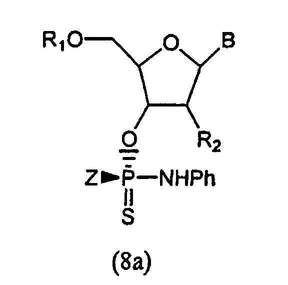 with sodium hydride or 1, 8-diazabicyclo[5.4.0]undec-7-ene and carbon dioxide to form a transient nucleoside 3'-O-(Z-substituted)phosphonothioic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7a) 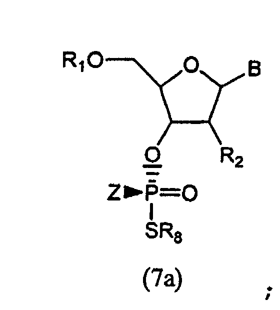 and
(c) reacting the alkylated nucleoside intermediate with a nucleoside of formula (5) 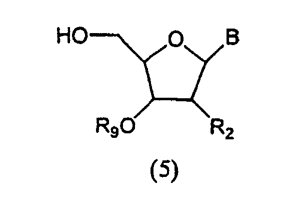 in presence of an activator and a lithium salt in an aprotic organic solvent to form the chirally pure dinucleoside of formula (6a).

In another embodiment of the invention a method for the synthesis of a chirally pure dinucleoside of formula (6a) 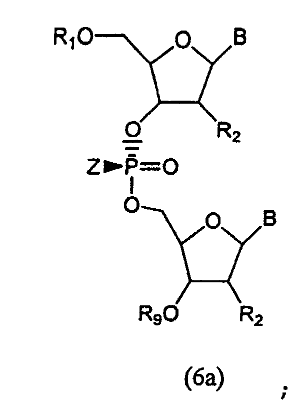 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10a) 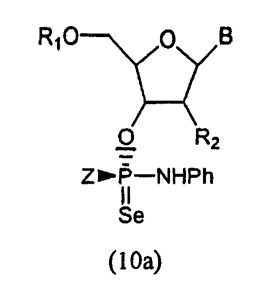 with sodium hydride or 1, 8-diazabicyclo[5.4.0]undec-7-ene and carbon dioxide to form a transient nucleoside 3'-O-(Z-substituted)phosphonoselenoic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11a) 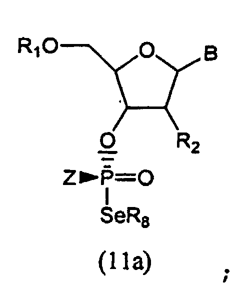 and
(c) reacting the alkylated nucleoside intermediate with a nucleoside of formula (5) 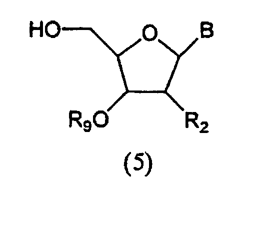 in presence of an activator and a lithium salt in an aprotic organic solvent to form the chirally pure dinucleoside of formula (6a).

In still another embodiment a method for the synthesis of a chirally pure dinucleoside of formula (6a) 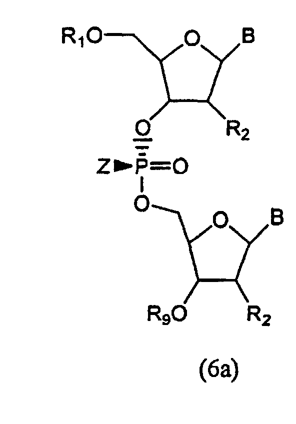 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8b) 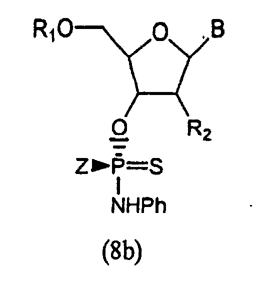 with potassium peroxymonosulfate or hydrogen peroxide to form a nucleoside intermediate of formula (9b) 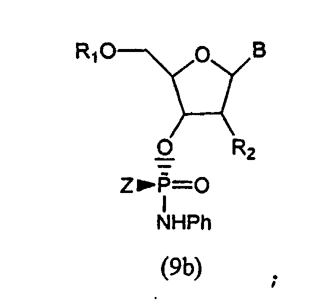
(b) reacting the nucleoside intermediate with sodium hydride or DBU and carbon disulfide to form a transient nucleoside 3'-O-(Z-substituted)phosphonothioic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7a) 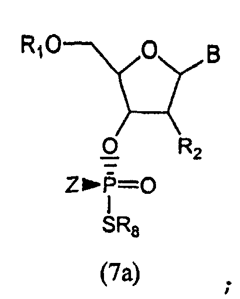 and
(d) reacting the alkylated nucleoside intermediate with a nucleoside of formula (5) 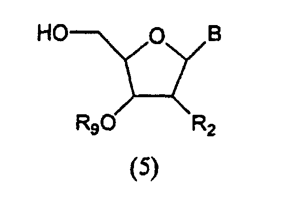 in presence of an activator and a lithium salt in an aprotic organic solvent to form the chirally pure dinucleoside of formula (6a).

In yet another embodiment of the invention a method for the synthesis of a chirally pure dinucleoside of formula (6a) 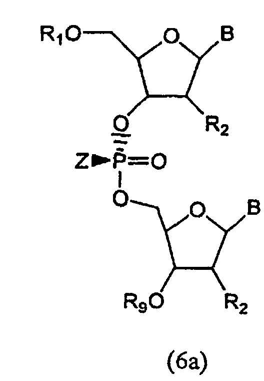 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10b) 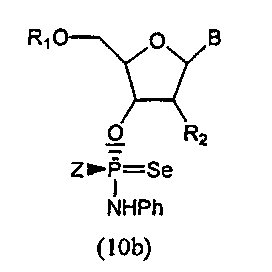 with potassium peroxymonosulfate or hydrogen peroxide to form a nucleoside intermediate of formula (9b) 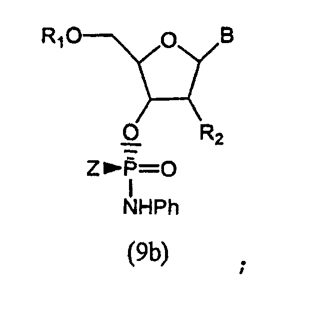
(b) reacting the nucleoside intermediate with sodium hydride or 1, 8-diazabicyclo[5.4.0]undec-7-ene and carbon diselenide to form a transient nucleoside 3'-O-(Z-substituted)phosphonoselenoic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form alkylated nucleoside intermediate of formula (11a) 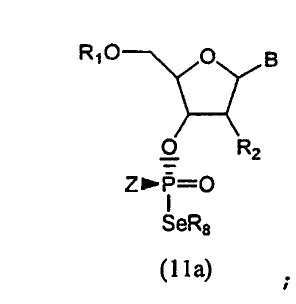 and
(d) reacting the alkylated nucleoside intermediate with a nucleoside of formula (5) 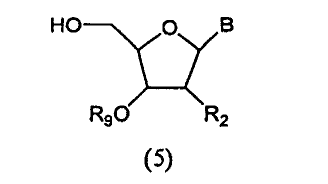 in presence of an activator and a lithium salt in an aprotic organic solvent to form the chirally pure dinucleoside of formula (6a).

In still another embodiment a method for the synthesis of a chirally pure dinucleoside of formula (6b) 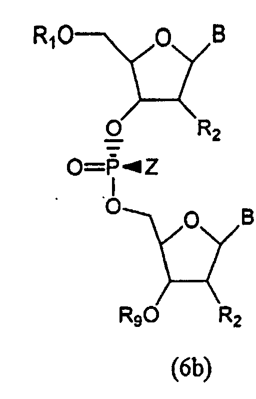 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8b) 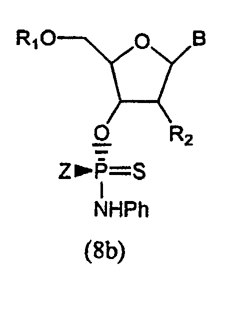 with sodium hydride or 1, 8-diazabicyclo[5.4.0]undec-7-ene and carbon dioxide to form a transient nucleoside 3'-O-(Z-substituted)phosphonothioic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7b) 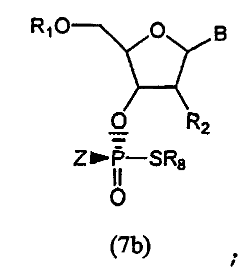 and
(c) reacting the alkylated nucleoside intermediate with a nucleoside of formula (5) 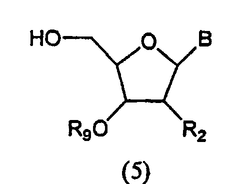 in presence of an activator and a lithium salt in an aprotic organic solvent to form the chirally pure dinucleoside of formula (6b).

In yet another embodiment a method for the synthesis of a chirally pure dinucleoside of formula (6b) 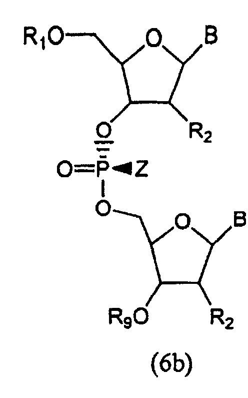 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10b) 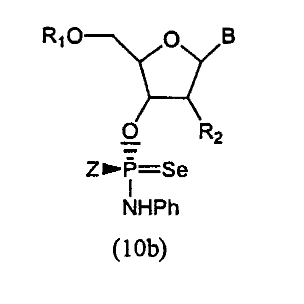 with sodium hydride or 1, 8-diazabicyclo[5.4.0]undec-7-ene and carbon dioxide to form a transient nucleoside 3'-O-(Z-substituted)phosphonoselenoic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11b) 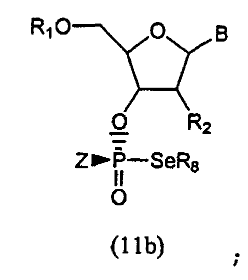 and
(c) reacting the alkylated nucleoside intermediate with a nucleoside of formula (5) 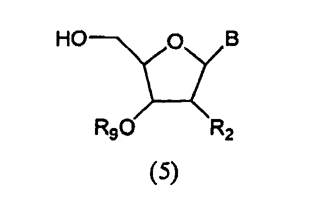 in presence of an activator and a lithium salt in an aprotic organic solvent to form the chirally pure dinucleoside of formula (6b).

In still another embodiment a method for the synthesis of a chirally pure dinucleoside of formula (6b) 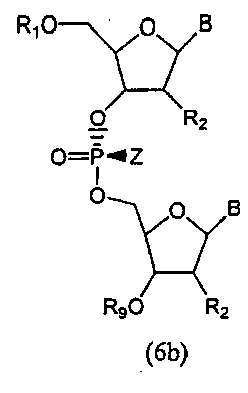 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8a) 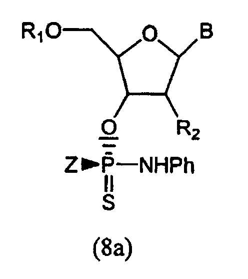 with potassium peroxymonosulfate or hydrogen peroxide to form a nucleoside intermediate of formula (9a) 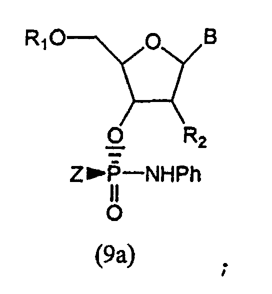
(b) reacting the nucleoside intermediate with sodium hydride or DBU and carbon disulfide to form a transient nucleoside 3'-O-(Z-substituted)phosphonothioic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7b) 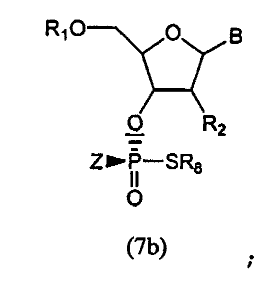 and
(d) reacting the alkylated nucleoside intermediate with a nucleoside of formula (5) 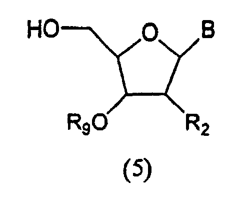 in presence of an activator and a lithium salt in an aprotic organic solvent to form the chirally pure dinucleoside of formula (6b).

In still another embodiment a method for the synthesis of a chirally pure dinucleoside of formula (6b) 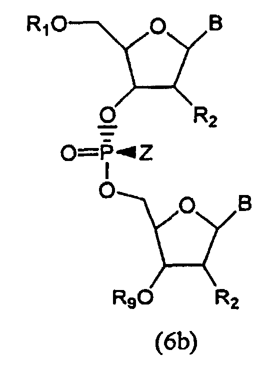 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10a) 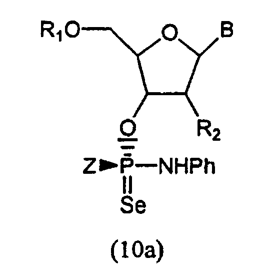 with potassium peroxymonosulfate or hydrogen peroxide to form a nucleoside intermediate of formula (9a) 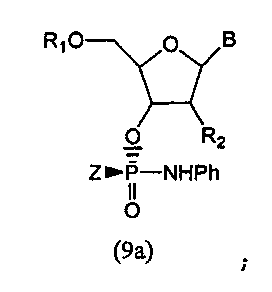
(b) reacting the nucleoside intermediate with sodium hydride or 1, 8-diazabicyclo[5.4.0]undec-7-ene and carbon diselenide to form a transient nucleoside 3'-O-(Z-substituted)phosphonoselenoic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11b) 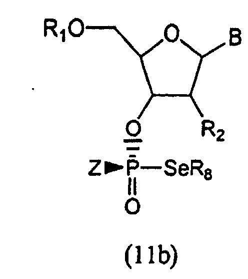 and
(d) reacting the alkylated nucleoside intermediate with a nucleoside of formula (5) 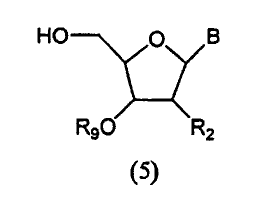 in presence of an activator and a lithium salt in an aprotic organic solvent to form the chirally pure nucleoside of formula (6b).

It is preferred that the aprotic organic solvent is acetonitrile, and the activator be 1,8-diazabicyclo[5.4.0]undec-7-ene.

### Brief Description of the Figures

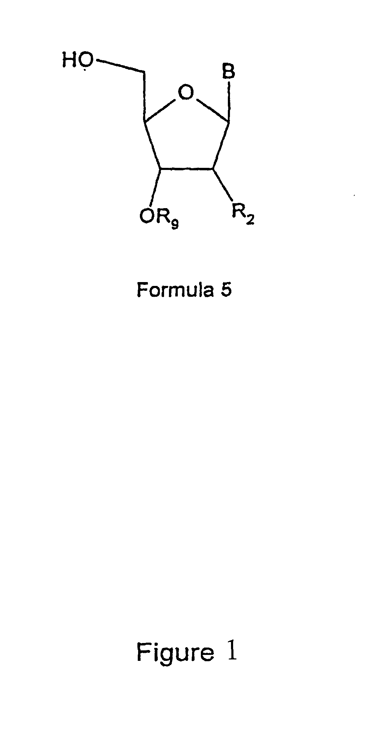

Figure 1 depicts the chemical structure of an organophosphorous mononucleoside derivative of formula (5).

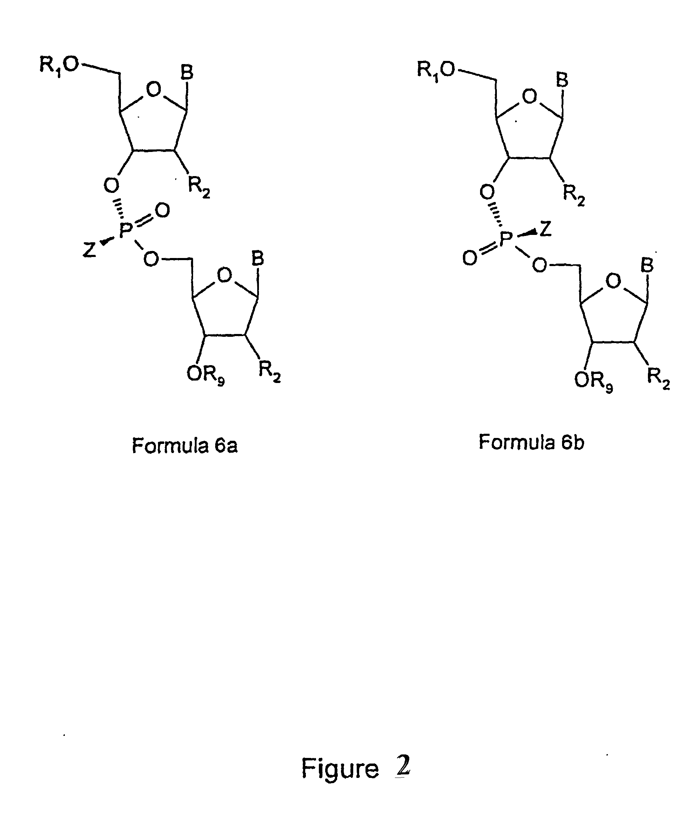

Figure 2 depicts the chemical structures of diastereomeric organophosphorous dinucleoside derivatives of formula (6a) and (6b).

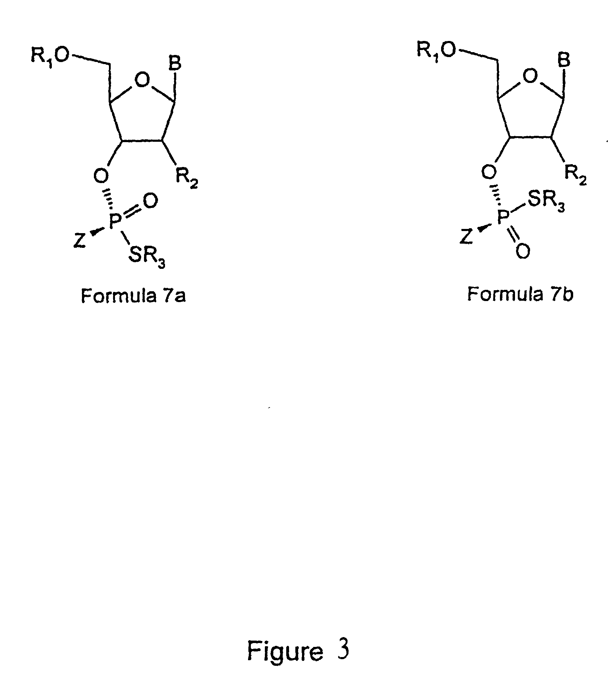

Figure 3 depicts the chemical structures of diastereomeric organophosphorous dinucleoside derivatives of formula (7a) and (7b).

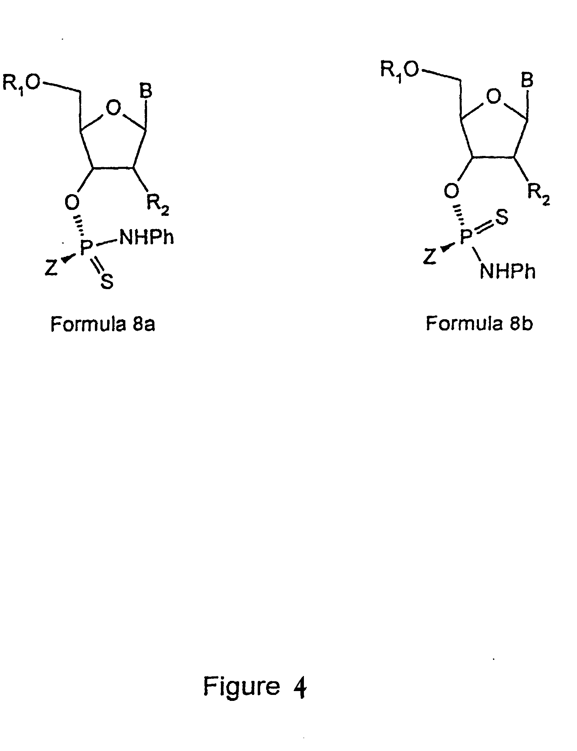

Figure 4 depicts the chemical structures of diastereomeric organophosphorous dinucleoside derivatives of formula (8a) and (8b).

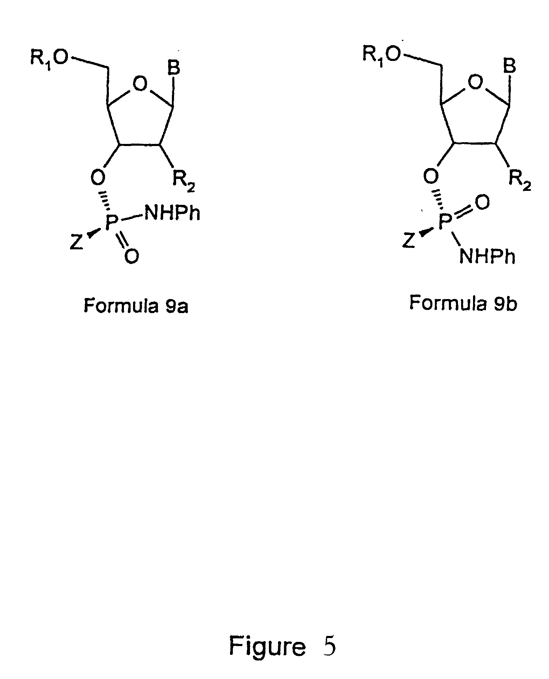

Figure 5 depicts the chemical structures of diastereomeric organophosphorous dinucleoside derivatives of formula (9a) and (9b).

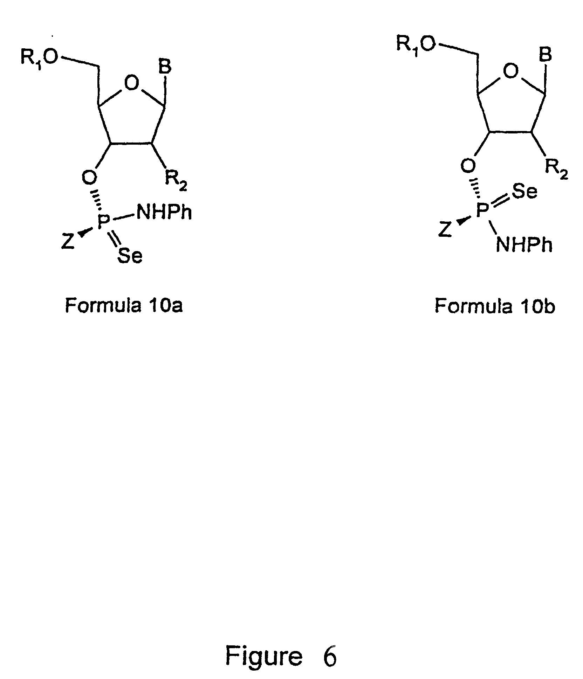

Figure 6 depicts the chemical structures of diastereomeric organophosphorous dinucleoside derivatives of formula (10a) and (10b).

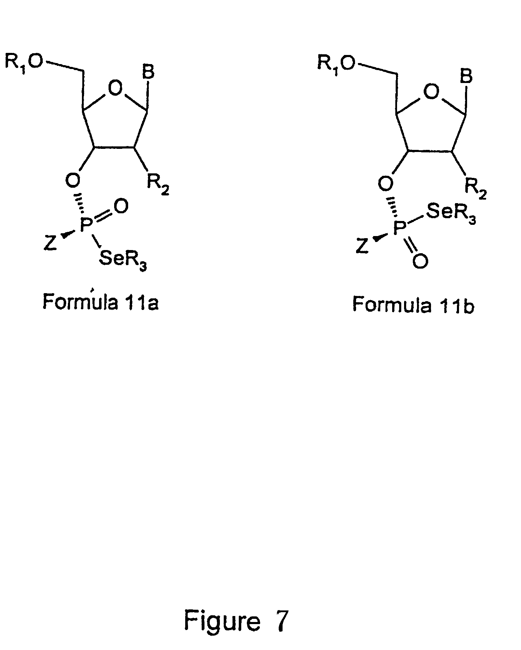

Figure 7 depicts the chemical structures of diastereomeric organophosphorous dinucleoside derivatives of formula (11a) and (11b).

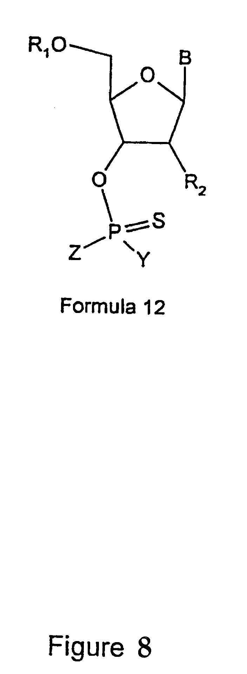

Figure 8 depicts the chemical structure of an organophosphorous dinucleoside derivative of formula (12).

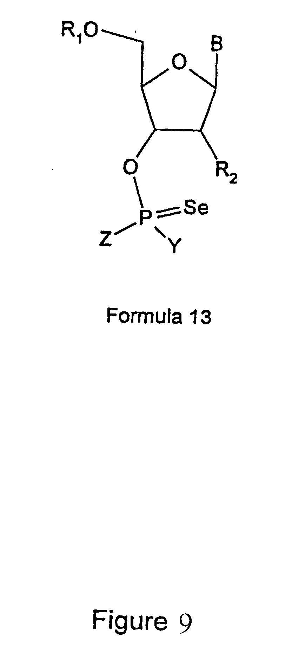

Figure 9 depicts the chemical structure of an organophosphorous dinucleoside derivative of formula (13).

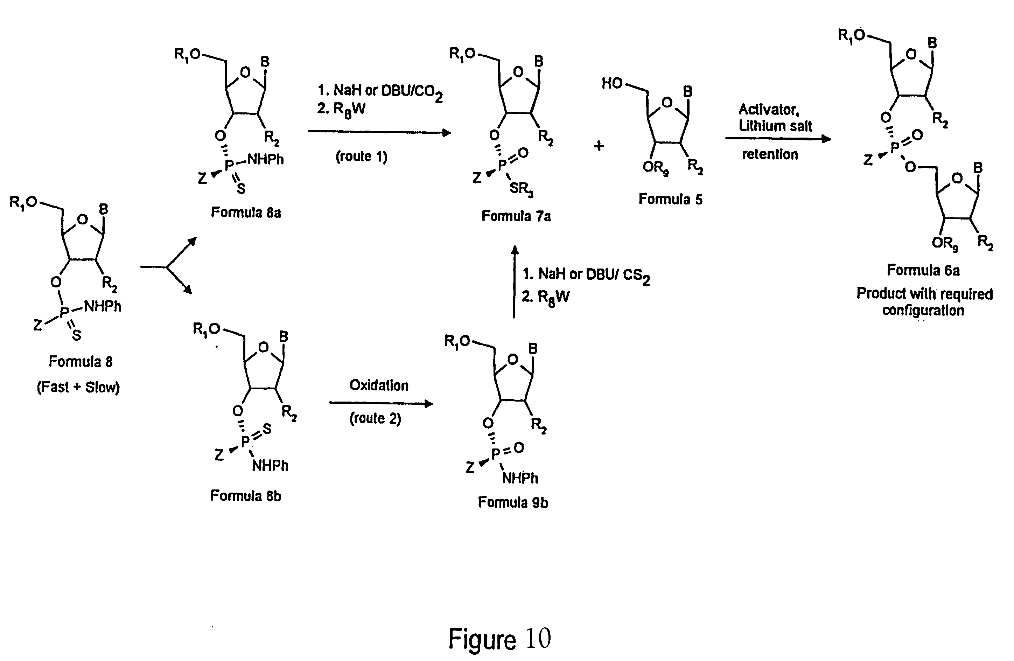

Figure 10 depicts a reaction scheme for a method for the synthesis of an organophosphorus dinucleotide of formula (6a) from diastereomeric organophosphorus derivatives (8a) and (8b).

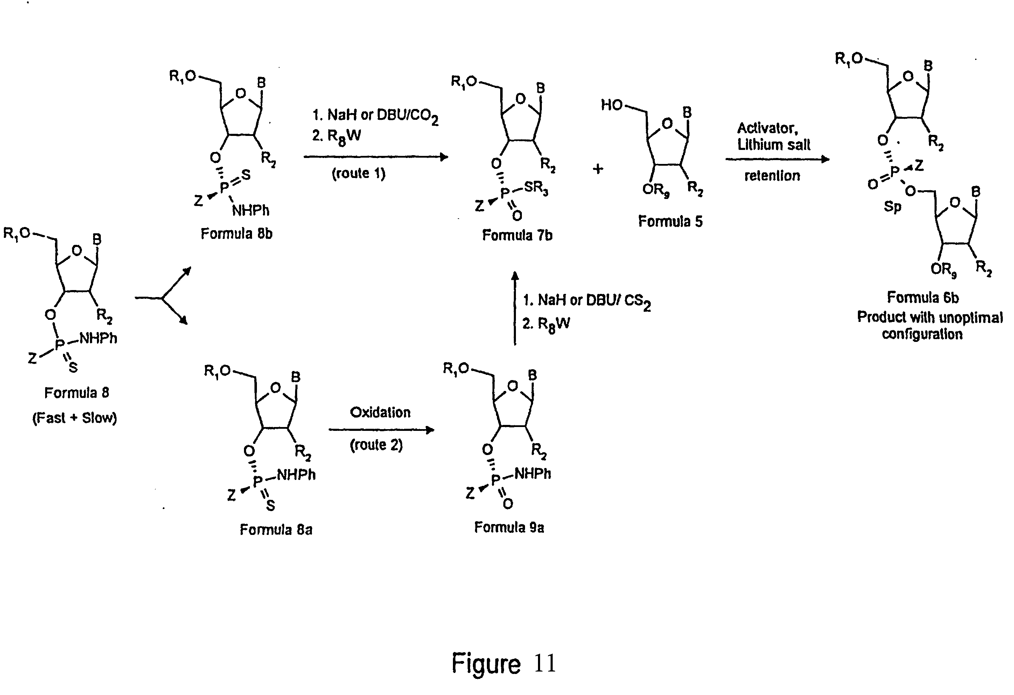

Figure 11 depicts a reaction scheme for a method for the synthesis of an organophosphorus dinucleotide of formula (6b) from diastereomeric organophosphorus derivatives (8a) and (8b).

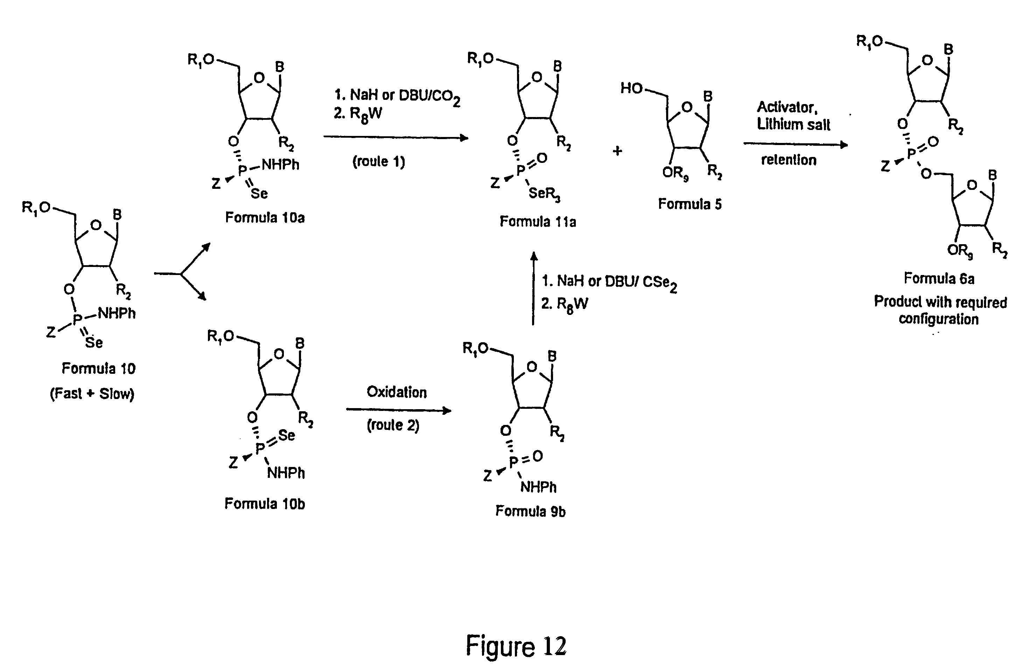

Figure 12 depicts a reaction scheme for a method for the synthesis of an organophosphorus dinucleotide of formula (6a) from diastereomeric organophosphorus derivatives (10a) and (10b).

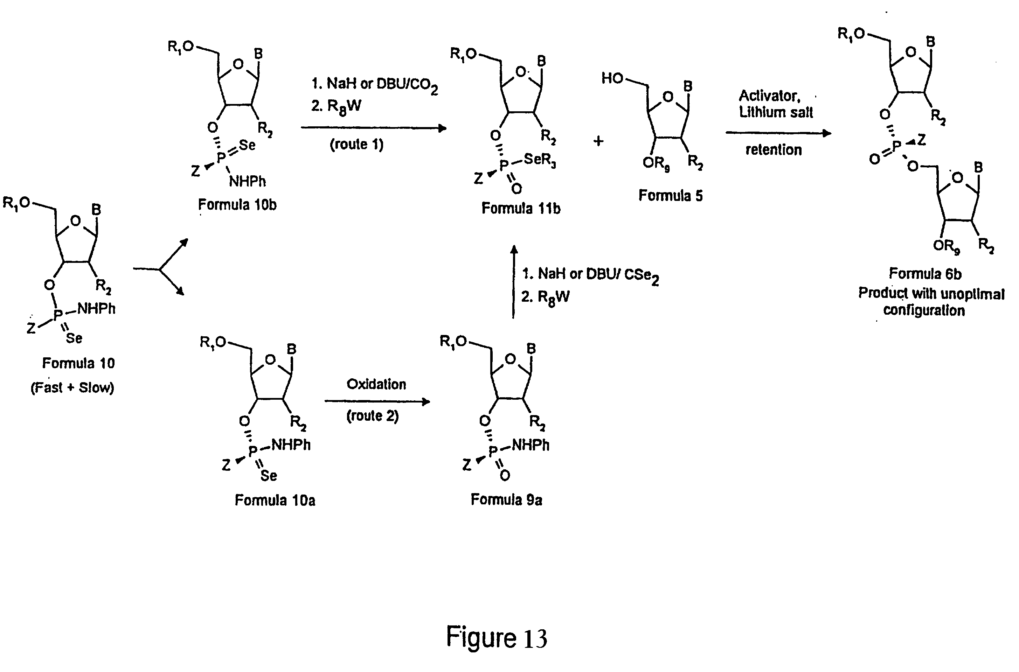

Figure 13 depicts a reaction scheme for a method for the synthesis of organophosphorus dinucleotide of formula 6b from diastereomeric organophosphorus derivatives 10a and 10b.

### Detailed Description

Prior to setting forth the invention, it may be helpful to an understanding thereof to first set forth definitions of certain terms that will be used hereinafter. These terms have the following meaning unless expressly stated to the contrary.

The term "alkyl" refers to saturated aliphatic groups including straight-chain, branched-chain and cyclic groups. Suitable alkyl groups include cyclohexyl and cyclohexylmethyl. "Lower alkyl" refers to alkyl groups of 1 to 6 carbon atoms.

The term "aryl" refers to aromatic groups which have at least one ring having a conjugated pi electron system and includes carbocyclic aryl, heterocyclic aryl and biaryl groups, all of which may be optionally substituted.

The term "carbocyclic aryl" refers to aromatic groups wherein the ring atoms on the aromatic ring are carbon atoms. Carbocyclic aryl groups include monocyclic carbocyclic aryl groups and naphthyl groups, all of which may be optionally substituted. Suitable carboxyclic aryl grouyps include phenyl and naphthyl.

The term "aromatic heterocycle" refers to aromatic groups having from 1 to 3 heteroatoms as ring atoms in the aromatic ring and the remainder of the ring atoms are carbon atoms. Suitable heteroatoms include oxygen, sulfur, and nitrogen, and suitable heterocyclic aryls include furanyl, thienyl, poyridyl, pyrroilyl, pyrimidyl, pyrazinyl, imidazolyl, and the like.

The term "biaryl" refers to phenyl substituted by carbocyclic or heterocyclic aryl as defined herein, ortho, meta or para to the point of attachment of the phenyl ring, advantageously para.

The term "lower" referred to herein in connection with organic radicals or compounds defines such with up to and including 6, preferably up to and including 4 and advantageously one or two carbon atoms. Such groups may be straight chain or branched chain.

The term "alkoxy" refers to -OR wherein R is alkyl.

The term "aralkyl" refers to an alkyl group substituted with an aryl group. Suitable aralkyl groups include benzyl, picolyl, and the like, all of which may be optionally substituted.

The term "cycloalkyl" refers to a cyclic alkyl group. Suitable cycloalkyl groups include cyclohexyl.

The term "alkenyl" refers to an unsaturated aliphatic group having at least one double bond.

The term "alkylene" refers to a divalent straing chain or branched chain saturated aliphatic radical.

The term "nucleoside base" refers to adenine, guanine, cytosine, thymidine, uracil as well as analogs and modified forms of naturally-occurring bases, including the pyrimidine-analogs such as pseudoisocytosine and pseudouracil, and other modified bases such as 8-substituted purines.

The term "Z-substituted" refers to a reagent or reactant which contains the Z substituent wherein Z is defined as aralkyl moiety, a haloalkyl moiety, an alkenyl moiety, an alkynyl moiety, or an alkaryl moiety of 1 to about 20 carbon atoms, an aminomethyl, or an aminoethyl and the reagent or reactant is that specified, for example, dichlorophosphine, phosphonothioic acid, phosphonoselenoic acid, phosphonoanilidoselenoate, or phosphonoanilidothioate.

The term "nucleoside" use in the terms "mononucleoside", "dinucleoside", and oligonucleoside refers to a subunit of a nucleic acid which comprises a 5-carbon sugar and a nitrogen-containing base. The term includes not only those nucleosidyl units having adenine, guanine, cytosine, thimidine and uracil, as their bases but also analogs and modified forms of naturally-occurring bases, including the pyrimidine analogs such as pseudoisocytosine and pseudouracil, and other modified bases such as 8-substituted purines. In RNA, the 5-carbon sugar is ribose; in DNA the 5-carbon sugar is deoxyribose. The term nucleoside also includes other analogs of such subunits, including those which have modified sugars such as 2'-O-alkyl ribose for example. The prefix of "mono", "di", and "oligo" refer to the number of nucleosides present. "Mono" means one and refers to a single nucleoside, "di" means two and refers to a compound comprising two nucleosides, and, "oligo" means many and refers to a compound with multiple nucleosides.

The limitations of the available methods for modification and synthesis of the organophosphorus derivatives have led to a continued need for other modifications which provide resistance to nucleases and satisfactory hybridization properties for antisense oligonucleotide diagnostics, therapeutics, and research. All references which have been cited below are hereby incorporated by reference in their entirety.

The organophosporus derivatives of this invention can be used in preparing oligonucleotides useful for diagnostics, therapeutics, as research reagents and for us in kits.

Chirally pure organophosphorus derivatives may be used in synthesizing oligonucleosides of preselected chirality, either enriched for R_{P} configuration, S _{F} configuration or a mixture thereof.

In particular, organophosphorus dinucleoside derivatives of the present invention of a defined chirality at the phosphonate may be coupled together using an automated DNA synthesizer. The dimer synthons have coupling groups which allow them to be coupled together to give a chirally enriched phosphonate oligomer (see Examples 5 to 13). From a stock of prepared organophosphorus dinucleoside derivatives, oligonucleosides of any nucleoside base sequence may be synthesized by linking together the appropriate dinucleosides. Dinucleosides are added to the growing oligonucleoside chain until an oligonucleoside having the desired number of nucleosides is obtained. The resulting oligonucleoside has a defined chirality at every other linkage.

Since the oligonucleotides thus produced may form duplexes or triple helix complexes or other forms of stable association with transcribed regions of nucleic acids, they may be used to interfere or inhibit or alter expression of a particular gene or target sequence in a living cell, allowing selective inactivation or inhibition or alteration of expression. The target sequence may be RNA, such as a pre-mRNA or an mRNA or DNA.

Many diseases and other conditions are characterized by the presence of undesired DNA or RNA, which may be in certain instances single stranded and in other instances double stranded. These diseases and conditions can be treated using the principles of antisense therapy as is generally understood in the art.

In accordance with the preferred embodiments, this invention is directed to methods for the synthesis of organophosphorus mononucleoside and dinucleoside derivatives.

A variety of methods are provided for the synthesis of diastereomeric mixtures of P-chiral dinucleotides. One synthesis method for the preparation of dinucleotides of formulas (6a) and (6b) comprises the steps;
(a) reacting nucleosides of formulas (8a) and (8b) with sodium hydride or 1,8-diazabicyclo[5.4.0]undec-7-ene and carbon dioxide to form transient nucleoside 3'-O-(Z-substituted)phosphonothioic acids,
(b) reacting the transient nucleosides from step (a) with an alkylating agent of the formula R₈W, wherein R₈ is CH₂C₆H₄R₇, wherein R₇ is H, Cl, or NO₂, and W is Cl, Br, or I, to form nucleoside intermediates of formulas (7a) and (7b)
(c) reacting the nucleoside intermediates with a nucleoside of formula (5) in presence of an activator and a lithium salt in an aprotic organic solvent to form the diastereomeric mixture of P-chiral dinucleosides.

Another method for the synthesis of diastereomeric mixtures of P-chiral dinucleotides for preparing dinucleotides of formulas (6a) and (6b) comprises the steps;
(a) reacting nucleosides of formulas (10a) and (10b) with sodium hydride or 1,8-diazabicyclo[5.4.0]undec-7-ene and carbon dioxide to form transient nucleoside 3'-O-(Z-substituted)phosphonoselenoic acids,
(b) reacting the transient nucleosides with an alkylating agent of the formula R₈W, wherein R₈ is CH₂C₆H₄R₇, wherein R₇ is H, Cl, or NO₂, and W is Cl, Br, or I, to form nucleoside intermediates of formulas (11a) and (11b),
(c) reacting the nucleoside intermediates with a nucleoside of formula (5) in presence of an activator and a lithium salt in an aprotic organic solvent to form the diastereomeric mixture of P-chiral dinucleosides.

Another method for the synthesis of diastereomeric mixtures of P-chiral dinucleotides for preparing dinucleotides of formulas (6a) and (6b) comprises the steps;
(a)reacting nucleosides of formulas (10a) and (10b) with sodium hydride or 1,8-diazabicyclo[5.4.0]undec-7-ene and carbon dioxide to form transient nucleoside 3'-O-(Z-substituted)phosphonoselenoic acids,
(b) reacting the transient nucleosides with an alkylating agent of the formula R₈W, wherein R₈ is CH₂C₆H₄R₇, wherein R₇ is H, Cl, or NO₂, and W is Cl, Br, or I, to form nucleoside intermediates of formulas (11a) and (11b),
(c) reacting the nucleoside intermediates with a nucleoside of formula (5) in presence of an activator and a lithium salt in an aprotic organic solvent to form the diastereomeric mixture of P-chiral dinucleosides.

Still another method for the synthesis of dinucleotides of formulas (6a) and (6b) comprises the steps;
(a) reacting a nucleoside of formula (10a) and (10b) with sodium hydride or 1,8-diazabicyclo[5.4.0]undec-7-ene and carbon dioxide to form transient nucleoside 3'-O-(Z-substituted)phosphonoselenoic acids,
(b) reacting the transient nucleosides with an alkylating agent of the formula R₈W, wherein R₈ is CH₂C₆H₄R₇, wherein R₇ is H, Cl, or NO₂, and W is Cl, Br, or I, to form nucleoside intermediates of formulas (11a) and (11b),
(c) reacting the nucleoside intermediates with a nucleoside of formula (5) in presence of an activator and a lithium salt in an aprotic organic solvent to form the diastereomeric mixture of P-chiral dinucleosides.

Yet another method for the synthesis of dinucleotides of formulas (6a) and (6b) wherein R₁ is a protecting group; R₂ is H, OH, or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety or an atoms, an aminomethyl, or an aminoethyl; and B is an N-protected nucleoside base comprises the steps;
(a) reicting nucleosides of formulas (8a) and (8b) with potassium peroxymonosulfate or hydrogen peroxide to form.intermediate nucleosides of formulas (9a) and (9b),
(b) reacting the intermediate nucleosides with sodium hydride or DBU and carbon disulfide to form transient nucleoside 3'-O-(Z-substituted)phosphonothioic acids,
(c) reacting the transient nucleosides with an alkylating agent of the formula R₈W, wherein R₈ is CH₂C₆H₄R₇, wherein R₇ is H, Cl, or NO₂, and W is Cl, Br, or I, to form nucleoside intermediates of formulas (7a) and (7b),
(d) reacting the nucleoside intermediates from step (a) with a nucleoside of formula (5) in presence of an activator and a lithium salt in an aprotic organic solvent to form the diastereomeric mixture of P-chiral dinucleosides.

Still another method for the synthesis of P-chiral dinucleosides of formulas (6a) and (6b) wherein R₁ is a protecting group; R₂ is H, OH, or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety or an alkaryl moiety, or an aryl moiety of 1 to about 20 carbon atoms, an aminomethyl, or an aminoethyl; and B is an N-protected nucleoside base comprises the steps;
(a) reacting nucleosides of formulas (10a) and (10b) with potassium peroxymonosulfate or hydrogen peroxide to form the intermediate nucleosides of formulas (9a) and (9b),
(b) reacting the intermediate nucleosides with sodium hydride or DBU and carbon diselenide to form transient nucleoside 3'-O-(Z-substituted)phosphonoselenoic acids,
(c) reacting the transient nucleosides with an alkylating agent of the formula R₈W, wherein R₈ is CH₂C₆H₄R₇, wherein R₇ is H, Cl, or NO₂, and W is Cl, Br, or I, to form nucleoside intermediates of formulas (11a) and (11b),
(d) reacting the nucleoside intermediates with a nucleoside of formula (5) in presence of an activator and a lithium salt in an aprotic organic solvent to form the diastereomeric mixture of P-chiral dinucleosides.

A variety of synthesis methods are provided for the synthesis of chirally pure dinucleosides. One method for the synthesis of a chirally pure dinucleotide (6a) wherein R₁ is a protecting group; R₂ is H, OH, or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 .carbon atoms, an aminomethyl, or an aminoethyl; and B is an N-protected nucleoside base comprises the steps;
(a) reacting a nucleoside of formula (8a) with sodium hydride or 1,8-diazabicyclo[5.4.0]undec-7-ene and carbon dioxide to form a transient nucleoside 3'-O-(Z-substituted) phosphonothioic acid,
(b) reacting the transient nucleoside from step (a) with an alkylating agent of the formula R₈W, wherein R₈ is CH₂C₆H₄R₇, wherein R₇ is H, Cl, or NO₂, and W is Cl, Br, or I, to form a nucleoside intermediate of formula (7a),
(c) reacting the nucleoside intermediate from step (b) with a nucleoside of formula (5) in presence of an activator and a lithium salt in an aprotic organic solvent to form the chirally pure dinucleoside.

Another method for the synthesis of a chirally pure dinucleotide (6a) wherein R₁ is a protecting group; R₂ is H, OH, or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and B is an N-protected nucleoside base comprises the steps;
(a) reacting a nucleoside of formula (10a) with sodium hydride or 1,8-diazabicyclo[5.4.0]undec-7-ene and carbon dioxide to form a transient nucleoside 3'-O-(Z-substituted)phosphonoselenoic acid,
(b) reacting the transient nucleoside from step (a) with an alkylating agent of the formula R₈W, wherein R₈ is CH₂C₆H₄R₇, wherein R₇ is H, Cl, or NO₂, and W is Cl, Br, or I, to form a nucleoside intermediate of formula (11a),
(c) reacting the nucleoside intermediate from step (b) with a nucleoside of formula (5) in presence of an activator and a lithium salt in an aprotic organic solvent to form the chirally pure dinucleoside.

Still another method for the synthesis of a chirally pure dinucleotide (6a) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and (e) B is an N-protected nucleoside base comprises the steps;
(a) reacting a nucleoside of formula (8b) with potassium peroxymonosulfate or hydrogen peroxide to form an intermediate nucleoside of formula (9b),
(b) reacting the intermediate nucleoside from step (a) with sodium hydride or DBU and carbon disulfide to form a transient nucleoside 3'-O-(Z-substituted) phosphonothioic acid,
(c) reacting the transient nucleoside from step (b) with an alkylating agent of the formula R₈W, wherein R₈ is CH₂C₆H₄R₇, wherein R₇ is H, Cl, or NO₂, and W is Cl, Br, or I, to form a alkylated nucleoside of formula (7a),
(d) reacting the alkylated nucleoside from step (c) with a nucleoside of formula (5) in presence of an activator and a lithium salt in an aprotic organic solvent to form the chirally pure dinucleoside.

Yet another method for the synthesis of chirally pure dinucleotide (6a) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and (e) B is an N-protected nucleoside base comprises the steps;
(a) reacting a nucleoside of the formula (10b) with potassium peroxymonosulfate or hydrogen peroxide to form a intermediate nucleoside of formula (9b),
(b) reacting the intermediate nucleoside from step (a) with sodium hydride or DBU and carbon diselenide to form a transient nucleoside 3'-O-(Z-substituted) phosphonoselenoic acid,
(c) reacting the transient nucleoside from step (b) with an alkylating agent of the formula R₈W, wherein R₈ is CH₂C₆H₄R₇, wherein R₇ is H, Cl, or NO₂, and W is Cl, Br, or I, to form a alkylated nucleoside of formula (11a),
(d) reacting the alkylated nucleoside from step (c) with a nucleoside of formula (5) in presence of an activator and a lithium salt in an aprotic organic solvent to form the chirally pure dinucleoside.

A method for the synthesis of chirally pure dinucleotide (6b) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl or an aminoethyl; and (e) B is an N-protected nucleoside base comprises the steps:
(a) reacting a nucleoside of formula (8b) with sodium hydride or DBU and carbon dioxide to form a transient nucleoside 3'-O-(Z-substituted)phosphonothioic acid,
(b) reacting the transient nucleoside from step (a) with an alkylating agent of the formula R₈W, wherein R₈ is CH₂C₆H₄R₇, wherein R₇ is H, Cl, or NO₂, and W is Cl, Br, or I, to form a nucleoside of formula(7b),
(c) reacting the alkylated nucleoside from step (b) with a nucleoside of formula (5) in presence of an activator and a lithium salt in an aprotic organic solvent to form the chirally pure dinucleoside.

Another method for the synthesis of chirally pure dinucleotide (6b) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and (e) B is an N-protected nucleoside base comprises the steps;
(a) reacting a nucleoside of the formula (10b) with sodium hydride or DBU and carbon dioxide to form a transient nucleoside 3'-O-(Z-substituted)phosphonoselenoic acid,
(b) reacting the transient nucleoside from step (a) with an alkylating agent of the formula R₈W, wherein R₈ is CH₂C₆H₄R₇, wherein R₇ is H, Cl, or NO₂, and W is Cl, Br, or I, to form a nucleoside of formula (11b),
(c) reacting the alkylated nucleoside from step (c) with a nucleoside of formula (5) in presence of an activator and a lithium salt in an aprotic organic solvent to form the chirally pure dinucleoside.

Another method for the synthesis of chirally pure dinucleotide (6b) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and B is an N-protected nucleoside base comprises the steps;
(a) reacting a nucleoside of formula (8a) with potassium peroxymonosulfate or hydrogen peroxide to form an intermediate nucleoside of formula (9a),
(b) reacting the intermediate nucleoside from step (a) with sodium hydride or DBU and carbon disulfide to form a transient nucleoside 3'-O-(Z-substituted) phosphonothioic acid,
(c) reacting the transient nucleoside from step (b) with an alkylating agent of the formula R₈W, wherein R₈ is CH₂C₆H₄R₇, wherein R₇ is H, Cl, or NO₂, and W is Cl, Br, or I, to form a nucleoside intermediate of formula (7b),
(d) reacting the alkylated nucleoside intermediate from step (c) with a nucleoside of formula (5) in presence of an activator and a lithium salt in an aprotic organic solvent to form the chirally pure dinucleosides.

Yet another method for the synthesis of chirally pure dinucleotide (6b) wherein R₁ is a protecting group; R₂ is H or alkoxy moiety of 1 to about 10 carbon atoms; R₉ is an acyl protecting group, a coupling group, or a silyl protecting group; Z is an alkyl moiety, an aralkyl moiety, a haloalkyl moiety, an alkenyl moiety, an alkynyl moiety, an alkaryl moiety, or an aryl moiety of 1 to about 20 carbon atoms, an aminomethyl, or an aminoethyl; and B is an N-protected nucleoside base comprises the steps;
(a) reacting a nucleoside of formula (10a) with potassium peroxymonosulfate or hydrogen peroxide to form an intermediate nucleoside of the formula (9a),
(b) reacting the intermediate nucleoside with sodium hydride or DBU and carbon diselenide to form a transient nucleoside 3'-O-(Z-substituted)phosphonoselenoic acid,
(c) reacting the transient nucleoside with an alkylating agent of the formula R₈W, wherein R₈ is CH₂C₆H₄R₇, wherein R₇ is H, Cl, or NO₂, and W is Cl, Br, or I, to form a nucleoside intermediate of formula (11b),
(d) reacting the alkylated nucleoside intermediate from step (c) with a nucleoside of formula (5) in presence of an activator and a lithium salt in an aprotic organic solvent to form the chirally pure dinucleosides.

Preferably when the aprotic solvent used in the synthesis methods is acetonuitrile and the activator is 1,8-diazabicyclo[5.4.0]undec-7-ene.

To assist in understanding the present invention, the following examples are included which describe the results of a series of experiments. The following examples relating to this invention should not, of course, be construed as specifically limiting the invention and such variation of the invention, now known or later developed, which would be within the purview of one skilled in the art are considered to fall within the scope of the invention as described herein and hereinafter claimed.

According to the following examples are offered by way of illustration and not by way of limitation.

### EXAMPLES

### Example 1

### General Procedure for the Synthesis of Formula 8 (Z = CH₃)

To a solution of methyldichlorophosphine (0.29 g, 2.5 mmol) and triethylamine (0.55 g, 5.5 mmol), in THF, cooled to -40°C, was added slowly a solution of the appropriately protected nucleoside (1.0 mmol) in THF (10 mL). After 30 minutes the reaction mixture was allowed to warm to room temperature and aniline (0.28 g, 3.0 mmol) was added dropwise, followed by elemental sulfur. The reaction was followed by tlc and when complete the reaction mixture was diluted with chloroform and extracted with aqueous NaHCO₃ solution. The extracts were dried over anhydrous Na₂SO₄ and concentrated under reduced pressure to provide a crude product that was purified by flash chromatography on silica gel (230-400 mesh) using a mixture of heptane (10-20%) in chloroform. Appropriate fractions were combined and concentrated under reduced pressure to yield the desired product as a mixture of diastereomers.

### Example 2

### [Rp,Sp]-5'-O-DMT-Thymidine 3'-O-(Methylphosphonoanilidothioate)

The title compound mixture was prepared from 5'-O-DMT-thymidine (0.540 g, 1 mmol) following the general procedure described in Example 1 and was obtained as a solid white foam. Yield: 0.66g (93%). ³¹P NMR (CDCl₃) δ 79.44, 79.58; MS (FAB⁻) m/e 712.4 [(M-H)⁻].

### Example 3

### [Rp,Sp]-5'-O-DMT-N⁴-Benzoyl-2'-Deoxycytidine 3'-O-(Methylphosphonoanilidothioate)

The title compound mixture was prepared from 5'-O-DMT-N⁴-benzoyl-2'-deoxycytidine( 1 mmol) following the general procedure described in Example 1 and was obtained as a colorless foam. Yield: 70%. ³¹P NMR (CDCl₃/C₆D₆) δ 79.38, 79.74.

### Example 4

### [Rp,Sp]-5'-O-DMT-N⁶-Benzoyl-2'-Deoxyadenosine 3'-O-(Methylphosphonoanilidothiate)

The title compound mixture was prepared from 5'-O-DMT-N⁶-2'-deoxyadenosine (0.657 g, 1 mmol) ) following the general procedure described in Example 1 and was obtained as a colorless foam. Yield: 75%. ³¹P NMR (CDCl₃) δ 79.21, 79.60.

### Example 5

### [Rp,Sp]-5'-O-DMT-N²-Isobutyryl-2'-Deoxyguanosine 3'-O-(Methylphonoanilidothioate)

The title compound mixture was prepared from 5'-O-DMT-N²-Isobutyryl-2'-deoxyguanosine (0.640 g, 1 mmol) following the general procedure described in Example 1 and was obtained as a white foam. Yield: 0.71 g (98%). ³¹P NMR (CDCL₃) δ 79.65, 79.72.

### Example 6

### [Rp, Sp]-5'-O-DMT-2'-O-Methyl Uridine 3'-O-(Methylphosphonoanilidothioate)

The title compound mixture was prepared from 5'-O-DMT-2'-O-Methyl uridine (0.560 g, 1 mmol) following the general procedure described in Example 1 and was obtained as a colorless foam. Yield: 0.68 g (85%). ³¹P NMR (CDCl₃) δ 81.96, 81.38.

### Example 7

### Separation of Diastereomers of Formula 8

Separation of the Rp and Sp diastereomers of Formula 8 described in Examples 2 through 6 was carried out by flash chromatography on silica gel using a mixture of 10-20% heptane in chloroform as eluent.

**Table 1**

| Example/8 | Diastereomer* | ³¹P NMR (δ) |
|---|---|---|
| II; B = Thymine | FAST | 79.44 |
| | SLOW | 79.58 |
| III; B = Cytosine | FAST | 79.38 |
| | SLOW | 79.74 |
| IV; B = Adenine | FAST | 79.21 |
| | SLOW | 79.60 |
| V; B = Guanine | FAST | 79.65 |
| | SLOW | 79.72 |
| Vl; B = Uracil | FAST | 81.38 |
| | SLOW | 81.96 |

| | | |
|---|---|---|
| * Mobility on Silica gel (Kieselgel 60, 240-400 mesh); eluent CHCl₃/MeOH (95:5 v/v) | | |

### Example 8

### General Procedure for the Synthesis of 5'-O-DMT-(N-Protected) Nucleoside 3'-O-(S-Benzyl Methylphosphonothioates) (Formula 7a and 7b, Z=CH₃)

To a stirred solution of the corresponding nucleoside 3'-O-(methylphosphonoanilidothioate) 8 (1 mmol), in dry DMF (10mL) was added NaH (1.2 molar equivalents) in several portions. Stirring was continued until evolution of hydrogen had ceased. To the resulting slurry was introduced a stream of dry gaseous CO₂. The reaction progress was monitored by TLC. Benzyl bromide (5 mmol) was added to the reaction mixture. When the reaction was complete, solvents and excess benzyl bromide were removed by rotary evaporation. The solid residue was dissolved in CHCl₃ and washed with saturated aqueous NaHCO₃ solution, dried and concentrated. The crude product was purification by flash chromatography on silica gel using 0-5% ethanol in chloroform as eluent. When substrate 8 was reacted as a mixture of diastereomers, the purification process was combined with the separation process to provide pure diastereomers of 7a (SLOW), and 7b (FAST).

### Example 9

### 5'-O-DMT-Thymidine 3'-O-(S-Benzyl Methylphosphonothioate)

The title compound was prepared from 8 (B = Thymine, Z= CH₃) (0.712 g, 1 mmol) following the general procedures described in Example 8. Yield: 0.55 g (86%). ³¹P NMR (CDCl₃) δ 56.44, 55.94.

### Example 10

### 5'-O-DMT-N⁴-Benzoyl-2'-Deoxycytidine 3'-O-(S-Benzyl Methylphosphonothioate)

The title compound was prepared from 8 (B = Cytosine, Z= CH₃) (1 mmol), following the general procedures described in Example 8. Yield: 62%. ³¹P NMR (CDCl₃) δ 55.41, 55.21.

### Example 11

### 5'-O-DMT-N⁶-Benzoyl-2'-Deoxyadenosine 3'-O-(S-Benzyl Methylphosphonothioate)

The title compound was prepared from 8 (B = Adenine, Z= CH₃) (1 mmol), following the general procedure described in Example 8. Yield: 75%. ³¹P NMR (CH₂Cl₂/C₆D₆) δ 55.80, 55.21 ppm.

### Example 12

### 5'-O-DMT-N⁴-Isobutyryl-2'-Deoxyguanosine 3'-O-(S-Benzyl Methylphosphonothioate)

The title compound was prepared from 8 (B = guanine, Z = CH₃) ( 1 mmol) following the general procedure described in Example 8. Yield: 75%. ³¹P NMR (CDCl₃) δ 56.01, 55.85.

### Example 13

### Oxidation of (FAST) 5'-O-DMT-Thymidine 3'-O-(Methylphosphonothioanilidate) 8a to (FAST) 5'-O-DMT-Thymidine 3'-O-(Methanephosphonoanilidate) 9a Using Potasium Peroxymonosulfate.

To a solution of compound 8a (FAST) (0.072 g, 1 mmol) in a mixture of MeOH and THF was added an aqueous solution of Potasium Peroxymonosulfate (pH 6.7-7, 2mmols). After 10 minutes a solution of 10% aqueous Na₂S₂O₃ was added and the mixture was extracted with chloroform. The extracts were dried over anhydrous Ma₂SO₄ and concentrated. The crude product was purified by flash chromatography on silica gel using a mixture of 10-20% heptane in chloroform as eluent to afford 0.047 g (73%) of diastereomerically pure (Fast)-9a. ³¹P NMR (CDCl₃) δ 30.1. MS (FAB-) *m*/*e* 696.4 [(M-H)⁻].

### Example 14

### Oxidation of (SLOW) 5'-O-DMT-Thymidine 3'-O-(Methylphosphonothioanilidate) 8b to (SLOW) 5'-O-DMT-Thymidine 3'-O-(Methanephosphonoanilidate) Using Potasium Peroxymonosulfate

Conversion of 8b (SLOW) to 9b (SLOW) was carried out using procedures analogous to those described in Example 13. Yield: 70%; ³¹P NMR (CDCl₃) δ 29.89.

### Example 15

### Conversion of 5'-O-DMT-Thymidine 3'-O-Methanephosphonoanilidate 9 to 5'-O-DMT-Thymidine 3'-O-(S-Benzyl Methanephosphonothioate) 7

Compounds 9 (FAST or SLOW) were dried prior to reaction and then dissolved in THF (2 mL). To this solution was added 1,8-diazabicyclo[5.4.0]undec-7-ene (DBU) (0.02 mL) and the reaction mixture was stirred at ambient temperature for 45 minutes, followed by addition of CS₂ (1 mL). After 20 minutes benzyl bromide was added (5 equivalents) and the reaction progress was monitored by tlc. When the reaction was complete the mixture was diluted with water and extracted with chloroform. The extracts were dried over anhydrous Na₂SO₄ and concentrated. The crude product was purified by flash chromatography on silica gel using 0-5% ethanol in chloroform as eluent.

From compound 9a (FAST), product 7b (FAST) was obtained in 65% yield. ³¹P NMR (CDCl₃) δ 56.85. Compound 9b (SLOW) was converted to 7a (SLOW) in 70% yield. ³¹P NMR (CDCl₃) δ 55.38.

### Example 16

### General Procedure for the Synthesis of Dinucleoside(3',5')Methylphosphonates (FAST)-6a or (SLOW)-6b (Z = CH₃)

The corresponding diastereomerically pure compounds 7 (SLOW or FAST) (0.3 mmol) and 3'-O-acetyl (N-protected)-2'-deoxynucleoside 5 (0.1 mmol) were dried prior to reaction and then dissolved in dry pyridine (5 mL). To this solution was added lithium chloride (0.125 g, 3 mmol), followed by a solution of DBU (0.456 g, 3 mmol) in pyridine (1.5mL) in one portion. The reaction was stirred at room temperature and its progress was monitored by tlc. After the reaction was complete, solvent was evaporated and the oily residue was dissolved in chloroform and extracted with phosphate buffer. The organic extracts were dried over anhydrous Na₂SO₄ and concentrated. The crude product was purified by flash chromatography on silica gel using a mixture of 0-3% ethanol in chloroform as eluent.

### Example 17

### Synthesis of (FAST)-5'-O-DMT-2'-Deoxythymidylyl-(3'D5')-3-O-Acetyl-2'-Deoxythymidine 3'-Methylphosphonate

The title compound was prepared from the corresponding compound (SLOW)-7a following the general procedure described in Example 16. Yield: 85% ³¹P NMR (CDCl₃) δ 33.00. ¹H NMR (CDCl₃) δ 1.58 (d, J_{P-H} = 17.64 Hz, 3H, P-CH₃), MS (FAB⁻) *m*/*e* 887 [(M-H)⁻].

### Example 18

### (FAST)-N⁴-Benzoyl-5'-O-DMT-2'-Deoxycytidylyl-(3'D,5')-N⁴-Benzoyl-3'-O-Acetyl-2'-Deoxycytidine 3'-Methylphosphonate

The title compound was prepared from the corresponding compound (SLOW)-7a following the general procedure described in Example 16. Yield: 73%. ³¹P NMR (CDCl₃) δ 33.08. ¹H NMR (CDCl₃) δ 1.6 (d, J_{P-H} = 17.5 Hz, 3H, P-CH₃).
MS (FAB⁻) *m*/*e* 1067 [(M-H)⁻].

## Claims

1. A method for the synthesis of a diastereomeric mixture of P-chiral dinucleosides of formulas (6a) and (6b) 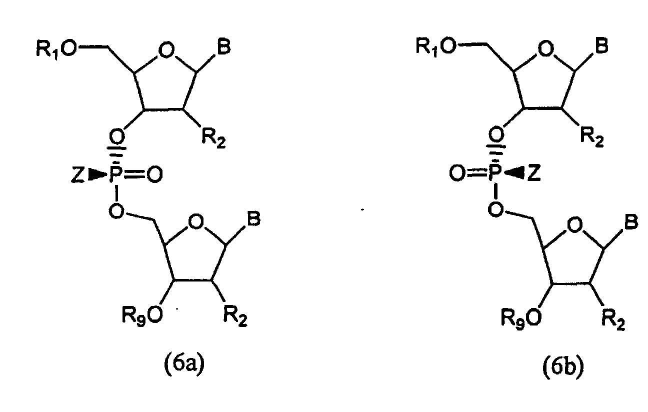 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (8a) and (8b) 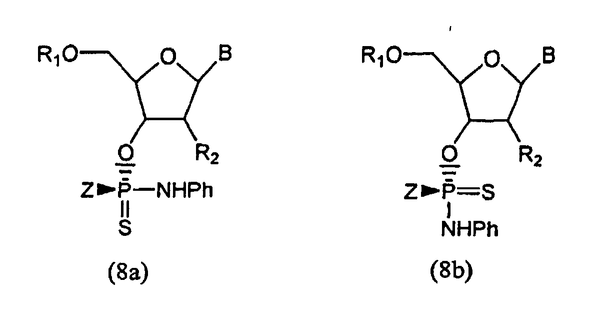 with sodium hydride or 1, 8-diazabicyclo[5.4.0]undec-7-ene and carbon dioxide to form transient nucleosides 3'-O-(Z-substituted)phosphonothioic acids;
(b) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (7a) and (7b) 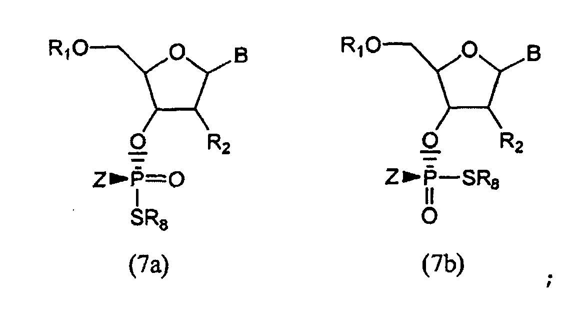 and
(c) reacting the alkylated nucleoside intermediates with a nucleoside of formula (5) 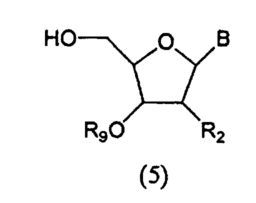 in presence of an activator and a lithium salt in an aprotic organic solvent to form the diastereomeric mixture of P-chiral dinucleosides of formulas (6a) and (6b).

2. A method for the synthesis of a diastereomeric mixture of P-chiral dinucleosides of formulas (6a) and (6b) 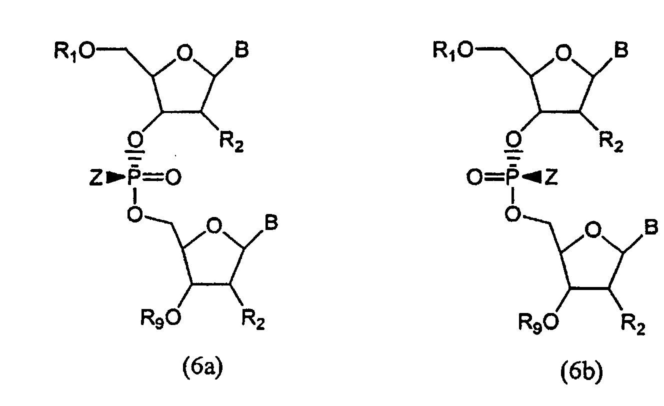 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (10a) and (10b) 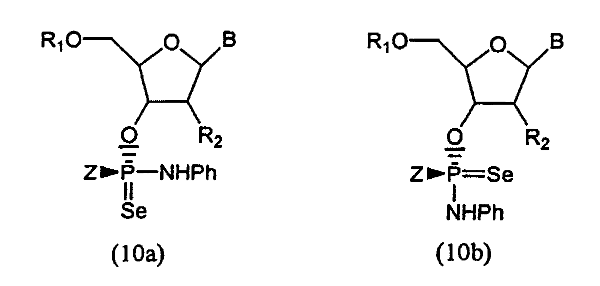 with sodium hydride or 1, 8-diazabicyclo[5.4.0]undec-7-ene and carbon dioxide to form transient nucleosides 3'-O-(Z-substituted)phosphonoselenoic acids;
(b) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (11a) and (11b) 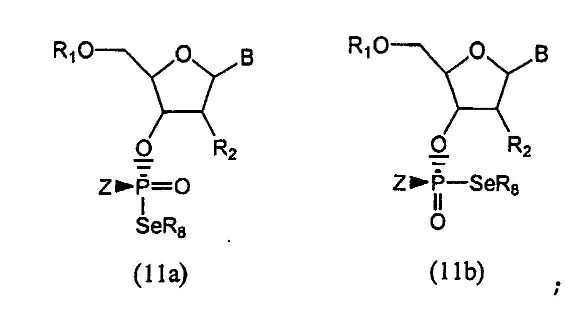 and
(c) reacting the alkylated nucleoside intermediates with a nucleoside of formula (5) 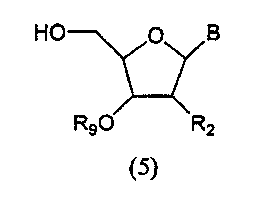 in presence of an activator and a lithium salt in an aprotic organic solvent to form the diastereomeric mixture of P-chiral dinucleosides of formulas (6a) and (6b).

3. A method for the synthesis of a diastereomeric mixture of P-chiral dinucleosides of formulas (6a) and (6b) 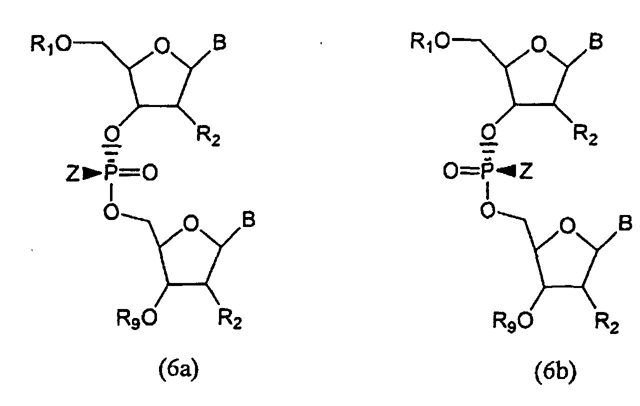 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formulas (8a) and (8b) 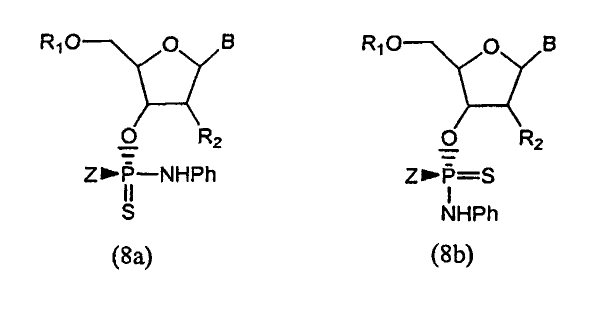 with potassium peroxymonosulfate or hydrogen peroxide to form nucleoside intermediates of formulas (9a) and (9b) 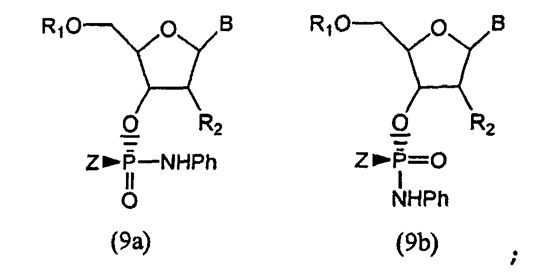
(b) reacting the nucleoside intermediates with sodium hydride or 1, 8-diazabicyclo[5.4.0]undec-7-ene and carbon disulfide to form transient nucleosides 3'-O-(Z-substituted)phosphonothioic acids;
(c) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (7a) and (7b) 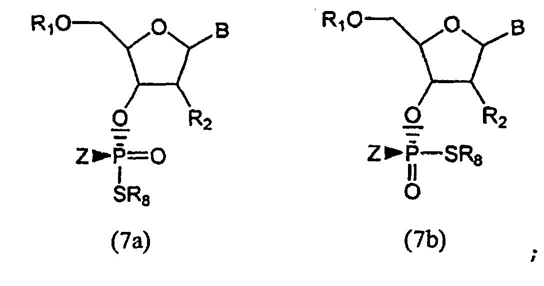 and
(d reacting the alkylated nucleoside intermediates with a nucleoside of formula (5) 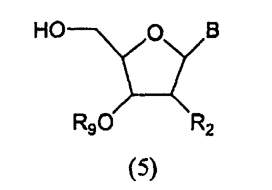 in presence of an activator and a lithium salt in an aprotic organic solvent to form the diastereomeric mixture of P-chiral dinucleosides of formulas (6a) and (6b).

4. A method for the synthesis of a diastereomeric mixture of P-chiral dinucleosides of formulas (6a) and (6b) 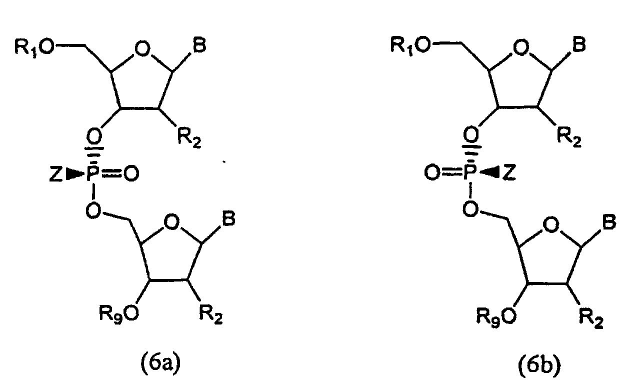 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting nucleosides of formula (10a) and (10b) 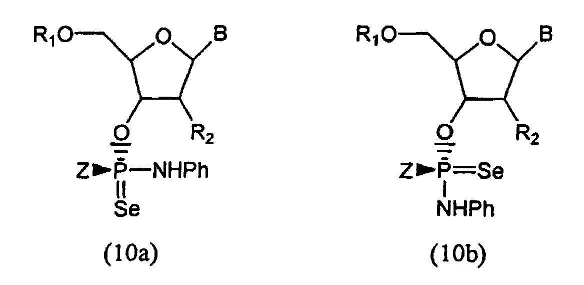 with potassium peroxymonosulfate or hydrogen peroxide to form nucleoside intermediates of formulas (9a) and (9b) 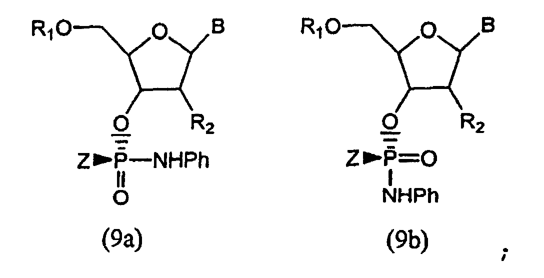
(b) reacting the nucleoside intermediates with sodium hydride or DBU and carbon diselenide to form transient nucleosides 3'-O-(Z-substituted)phosphonoselenoic acids;
(c) reacting the transient nucleosides with an alkylating agent of the formula R₈W, wherein R₈ is CH₂C₆H₄R₇, wherein R₇ is H, Cl, or NO₂, and W is Cl, Br, or I, to form alkylated nucleoside intermediates of formulas (11a) and (11b) 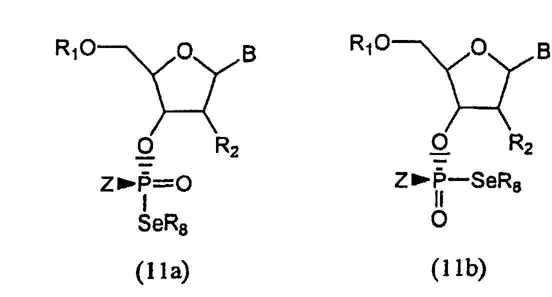 and
(d) reacting the alkylated nucleoside intermediates with a nucleoside of formula (5) 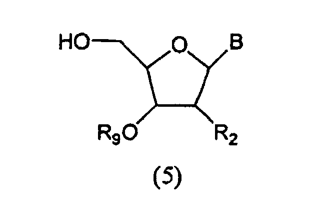 in presence of an activator and a lithium salt in an aprotic organic solvent to form the diastereomeric mixture of P-chiral dinucleosides of formulas (6a) and (6b).

5. A method for the synthesis of a chirally pure dinucleoside of formula (6a) 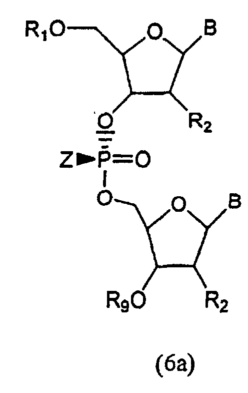 wherein
R₁ is a protecting group;
R₂ is H, OH, or alkoxy moiety of 1 to 10 carbon atoms;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8a) 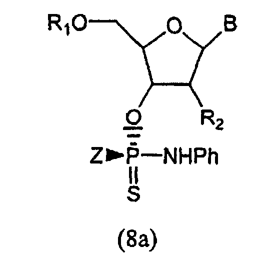 with sodium hydride or 1, 8-diazabicyclo[5.4.0]undec-7-ene and carbon dioxide to form a transient nucleoside 3'-O-(Z-substituted)phosphonothioic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7a) 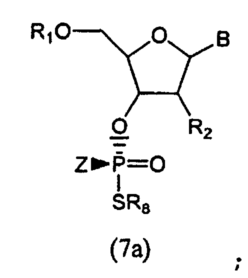 and
(c) reacting the alkylated nucleoside intermediate with a nucleoside of formula (5) 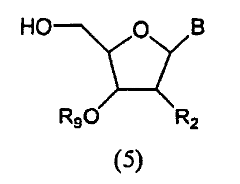 in presence of an activator and a lithium salt in an aprotic organic solvent to form the chirally pure dinucleoside of formula (6a).

6. A method for the synthesis of a chirally pure dinucleoside of formula (6a) 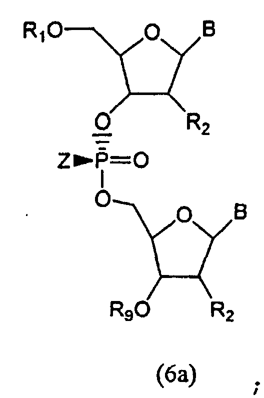 wherein
R₁ is a protecting group;
R₂ is H, OH, or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10a) 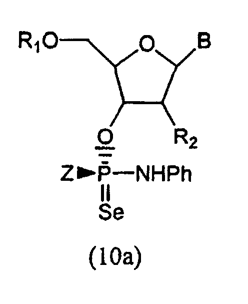 with sodium hydride or 1, 8-diazabicyclo[5.4.0]undec-7-ene and carbon dioxide to form a transient nucleoside 3'-O-(Z-substituted)phosphonoselenoic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11a) 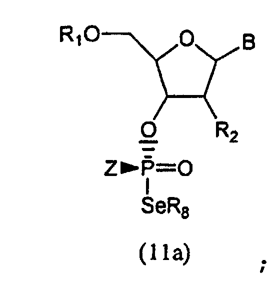 and
(c) reacting the alkylated nucleoside intermediate with a nucleoside of formula (5) 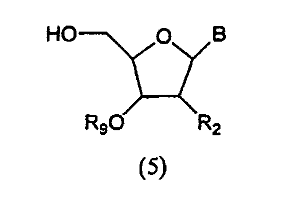 in presence of an activator and a lithium salt in an aprotic organic solvent to form the chirally pure dinucleoside of formula (6a).

7. A method for the synthesis of a chirally pure dinucleoside of formula (6a) 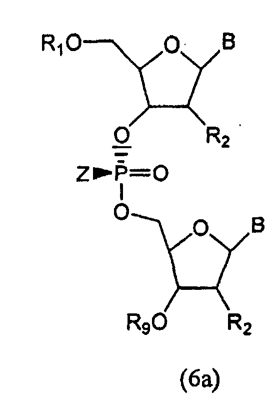 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8b) 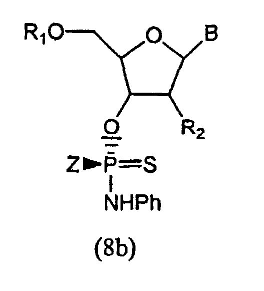 with potassium peroxymonosulfate or hydrogen peroxide to form a nucleoside intermediate of formula (9b) 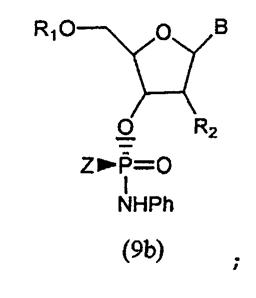
(b) reacting the nucleoside intermediate with sodium hydride or DBU and carbon disulfide to form a transient nucleoside 3'-O-(Z-substituted)phosphonothioic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7a) 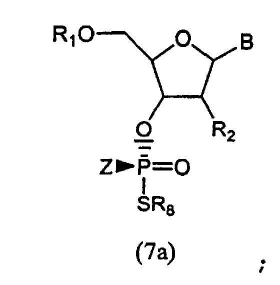 and
(d) reacting the alkylated nucleoside intermediate with a nucleoside of formula (5) 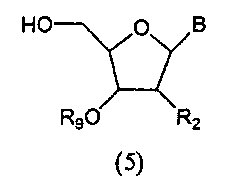 in presence of an activator and a lithium salt in an aprotic organic solvent to form the chirally pure dinucleoside of formula (6a).

8. A method for the synthesis of a chirally pure dinucleoside of formula (6a) 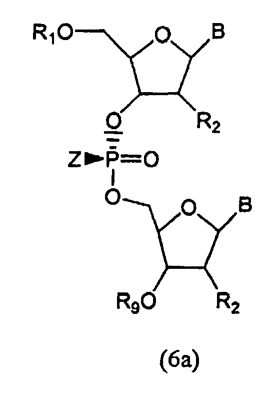 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10b) 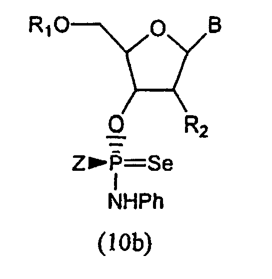 with potassium peroxymonosulfate or hydrogen peroxide to form a nucleoside intermediate of formula (9b) 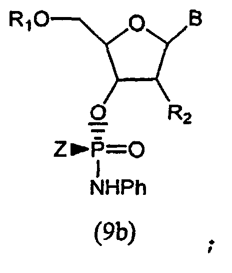
(b) reacting the nucleoside intermediate with sodium hydride or 1, 8-diazabicyclo[5.4.0]undec-7-ene and carbon diselenide to form a transient nucleoside 3'-O-(Z-substituted)phosphonoselenoic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form alkylated nucleoside intermediate of formula (11a) 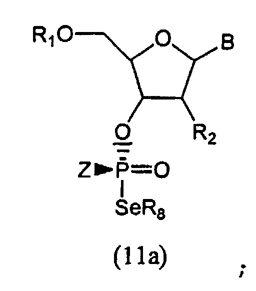 and
(d) reacting the alkylated nucleoside intermediate with a nucleoside of formula (5) 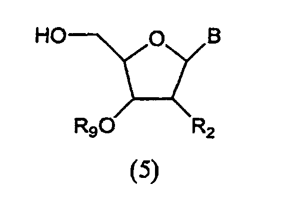 in presence of an activator and a lithium salt in an aprotic organic solvent to form the chirally pure dinucleoside of formula (6a).

9. A method for the synthesis of a chirally pure dinucleoside of formula (6b) 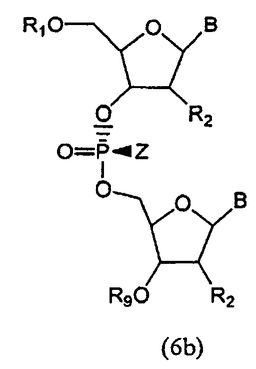 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8b) 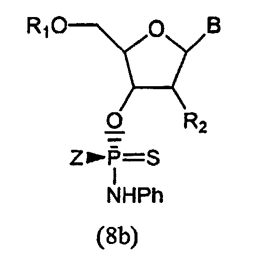 with sodium hydride or 1, 8-diazabicyclo[5.4.0]undec-7-ene and carbon dioxide to form a transient nucleoside 3'-O-(Z-substituted)phosphonothioic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7b) 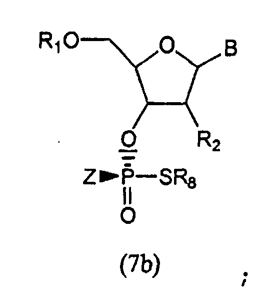 and
(c) reacting the alkylated nucleoside intermediate with a nucleoside of formula (5) 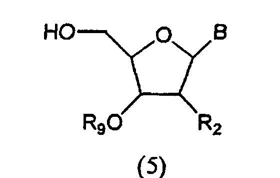 in presence of an activator and a lithium salt in an aprotic organic solvent to form the chirally pure dinucleoside of formula (6b).

10. A method for the synthesis of a chirally pure dinucleoside of formula (6b) 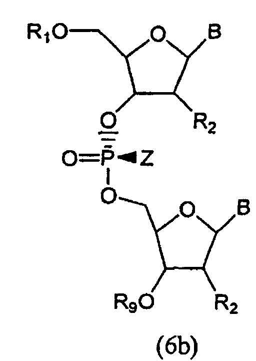 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10b) 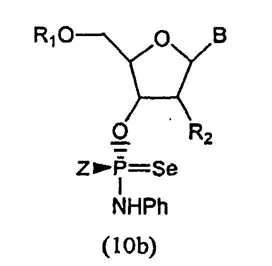 with sodium hydride or 1, 8-diazabicyclo[5.4.0]undec-7-ene and carbon dioxide to form a transient nucleoside 3'-O-(Z-substituted)phosphonoselenoic acid;
(b) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11b) 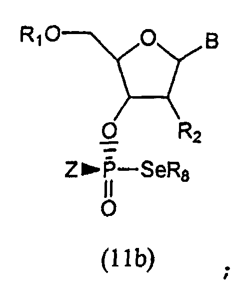 and
(c) reacting the alkylated nucleoside intermediate with a nucleoside of formula (5) 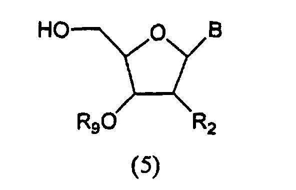 in presence of an activator and a lithium salt in an aprotic organic solvent to form the chirally pure dinucleoside of formula (6b).

11. A method for the synthesis of a chirally pure dinucleoside of formula (6b) 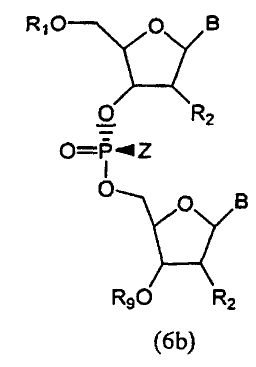 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (8a) 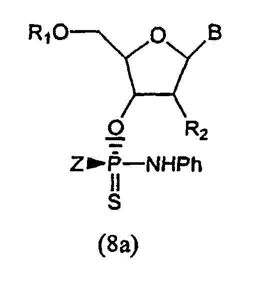 with potassium peroxymonosulfate or hydrogen peroxide to form a nucleoside intermediate of formula (9a) 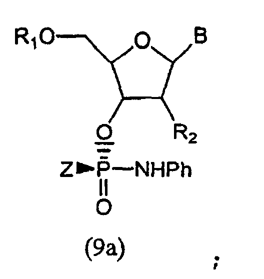
(b) reacting the nucleoside intermediate with sodium hydride or DBU and carbon disulfide to form a transient nucleoside 3'-O-(Z-substituted)phosphonothioic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (7b) 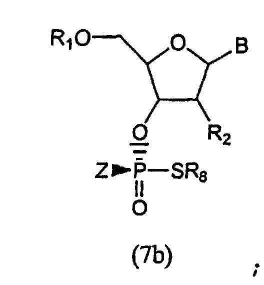 and
(d) reacting the alkylated nucleoside intermediate with a nucleoside of formula (5) 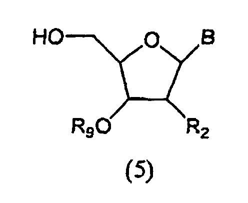 in presence of an activator and a lithium salt in an aprotic organic solvent to form the chirally pure dinucleoside of formula (6b).

12. A method for the synthesis of a chirally pure dinucleoside of formula (6b) 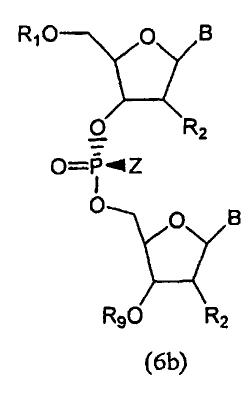 wherein
R₁ is a protecting group;
R₂ is H or alkoxy moiety of 1 to 10 carbons;
R₉ is an acyl protecting group, a coupling group, or a silyl protecting group;
Z is an alkyl moiety, an aralkyl moiety, a haloalkyl moiety, an alkenyl moiety, an alkynyl moiety, an alkaryl moiety, an aryl moiety of 1 to 20 carbons, an aminomethyl, or an aminoethyl;
B is an N-protected nucleoside base;
comprising the steps of:
(a) reacting a nucleoside of formula (10a) 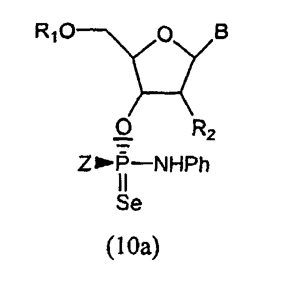 with potassium peroxymonosulfate or hydrogen peroxide to form a nucleoside intermediate of formula (9a) 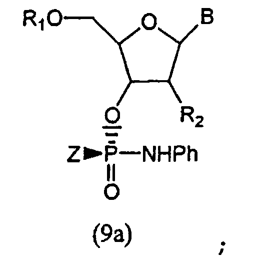
(b) reacting the nucleoside intermediate with sodium hydride or 1, 8-diazabicyclo[5.4.0]undec-7-ene and carbon diselenide to form a transient nucleoside 3'-O-(Z-substituted)phosphonoselenoic acid;
(c) reacting the transient nucleoside with an alkylating agent of the formula R₈W, wherein R₈ is CH₂C₆H₄R₇, wherein R₇ is H, Cl, or NO₂, and W is Cl, Br, or I, to form an alkylated nucleoside intermediate of formula (11b) 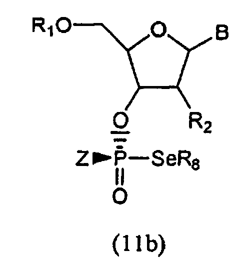 and
(d) reacting the alkylated nucleoside intermediate with a nucleoside of formula (5) 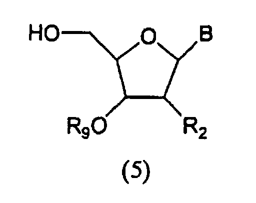 in presence of an activator and a lithium salt in an aprotic organic solvent to form the chirally pure nucleoside of formula (6b).

13. A method according to any of Claims 3 to 12 wherein the aprotic solvent is acetonitrile.

14. A method according to any of Claims 3 to 12 wherein the activator is 1, 8-diazabicyclo[5.4.0]undec-7-ene.

## Patentansprüche

1. Verfahren zur Synthese einer diastereomeren Mischung von P-chiralen Dinucleosiden der Formeln (6a) und (6b) 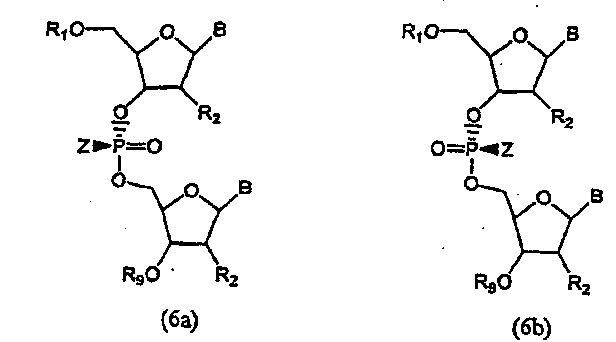 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (8a) und (8b) 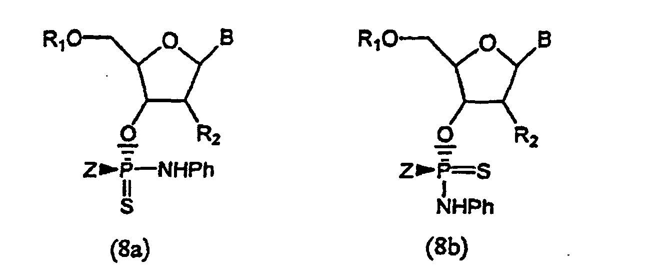 mit Natriumhydrid oder 1,8-Diazabicyclo[5.4.0]undec-7-en und Kohlenstoffdioxid, um die Übergangsnucleoside 3'-O-(Z-substituierte) Thiophosphonsäuren zu bilden;
(b) Reagieren der Übergangsnucleoside mit einem Alkylierungsmittel der Formel R₈W, wobei R₈ CH₂C₆H₄R₇ ist, wobei R₇ H, Cl oder NO₂ ist und W ist Cl, Br oder I, um alkylierte Nucleosidzwischenprodukte der Formeln (7a) und (7b) zu bilden 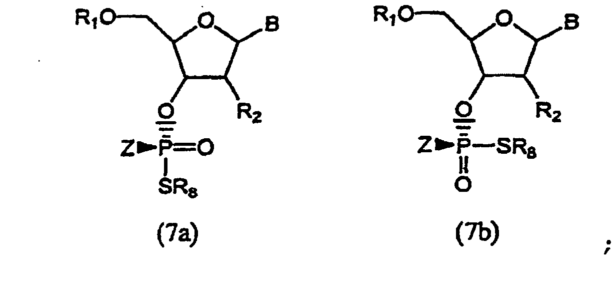 und
(c) Reagieren der alkylierten Nuceleosidzwischenprodukte mit einem Nucleosid der Formel (5) 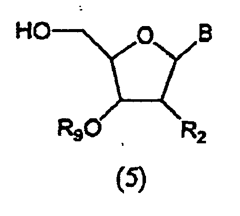 in Anwesenheit eines Aktivators und eines Lithiumsalzes in einem aprotischen organischen Lösungsmittel, um die diastereomere Mischung von P-chiralen Dinucleosiden der Formeln (6a) und (6b) zu bilden.

2. Verfahren zur Synthese einer diastereomeren Mischung von P-chiralen Dinucleosiden der Formel (6a) und (6b) 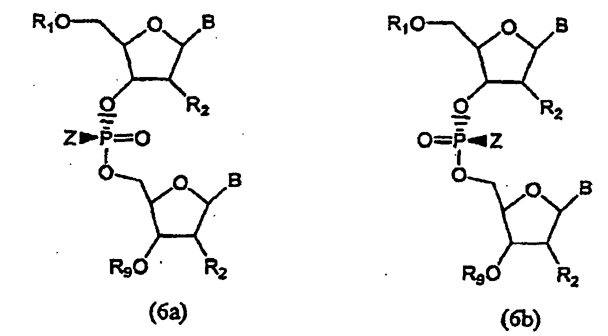 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formeln (10a) und (10b) 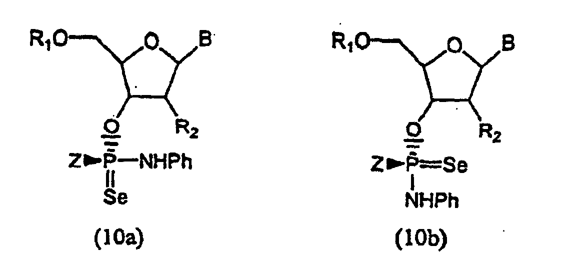 mit Natriumhydrid oder 1, 8-Diazabicyclo[5.4.0]undec-7-en und Kohlendioxid, um die Übergangsnucleoside 3'-O-(Z-substituierte)Selenophosphonsäuren zu bilden;
(b) Reagieren der Übergangsnucleoside mit einem Alkylierungsmittel der Formel R₈W, worin R₈ CH₂C₆H₄R₇ ist, wobei R₇ H, Cl oder NO₂ ist und W ist Cl, Br oder I, um alkylierte Nucleosidzwischenprodukte der Formeln (11a) und (11b) zu erhalten 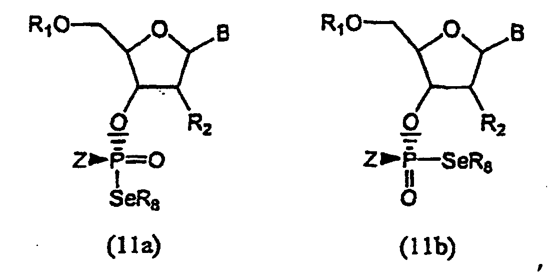 und
(c) Reagieren der alkylierten Nucleosidzwischenprodukte mit einem Nucleosid der Formel (5) 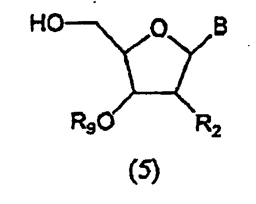 in Anwesenheit eines Aktivators und eines Lithiumsalzes in einem aprotischen organischen Lösungsmittels, um die diastereomere Mischung von P-chiralen Dinucleosiden der Formeln (6a) und (6b) zu bilden.

3. Verfahren zur Synthese einer diastereomeren Mischung von P-chiralen Dinucleosiden der Formeln (6a) und (6b) 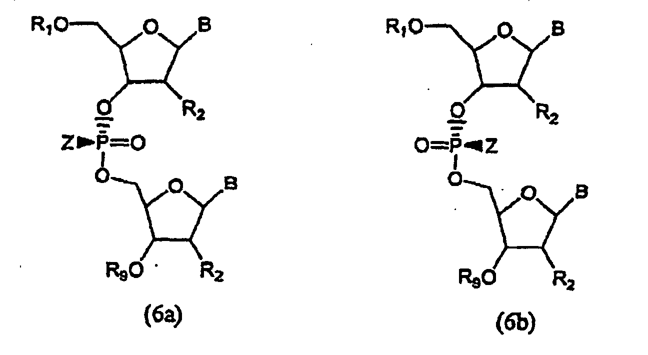 worin
R₁ eine Schutzgruppe ist;
R₂ ist H, OH oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formeln (8a) und (8b) 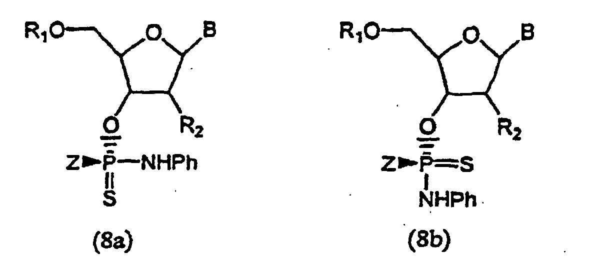 mit Kaliumperoxymonosulfat oder Wasserstoffperoxid, um Nucleosidzwischenprodukte der Formeln (9a) und (9b) zu bilden 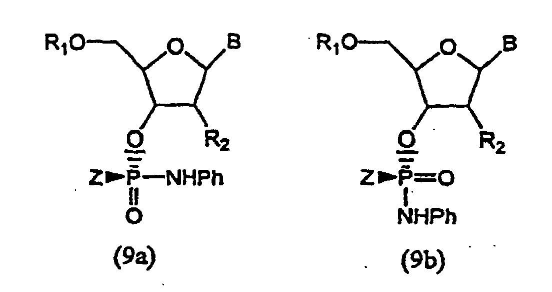
(b) Reagieren der Nucleosidzwischenprodukte mit Natriumhydrid oder 1,8-Diazabicyclo[5.4.0]undec-7-en und Kohlenstoffdisulfid, um Übergangsnucleoside 3'-O-(Zsubstituierte) Thiophosphonsäuren zu bilden;
(c) Reagieren der Übergangsnucleoside mit einem Alkylierungsmittel der Formel R₈W, wobei R₈ CH₂C₆H₄R₇ ist, wobei R₇ H, Cl oder NO₂ ist und W ist Cl, Br oder I, um alkylierte Nucleosidzwischenprodukte der Formeln (7a) und (7b) zu bilden 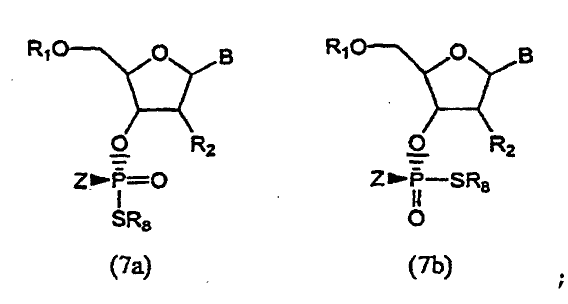 und
(d) Reagieren der alkylierten Nucleosidzwischenprodukte mit einem Nucleosid der Formel (5) 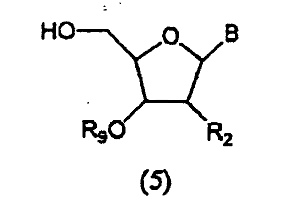 in Anwesenheit eines Aktivators und eines Lithiumsalzes in einem aprotischen organischen Lösungsmittel, um die diastereomere Mischung von P-chiralen Dinucleosiden der Formeln (6a) und (6b) zu bilden.

4. Verfahren zur Synthese einer diastereomeren Mischung von P-chiralen Dinucleosiden der Formeln (6a) und (6b) 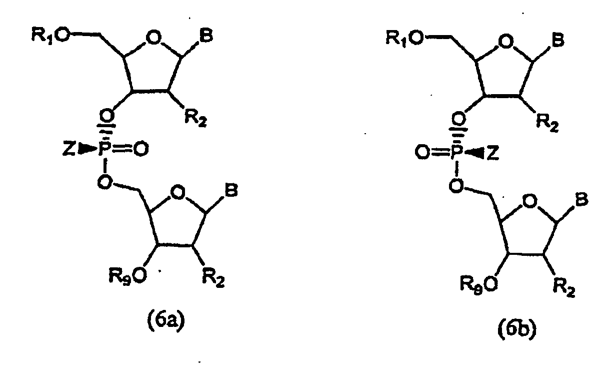 worin
R₁ eine Schutzgruppe ist;
R₂ ist H, OH oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formeln (10a) und (10b) 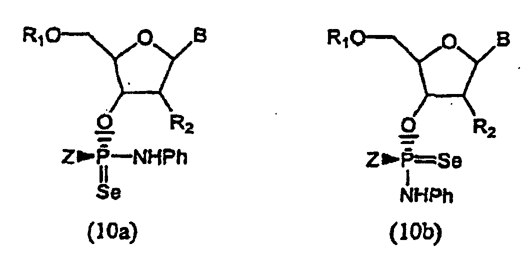 mit Kaliumperoxymonosulfat oder Wasserstoffperoxid, um die Nucleosidzwischenprodukte der Formeln (9a) und (9b) zu bilden 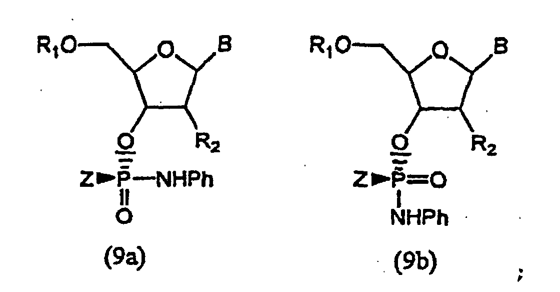
(b) Reagieren der Nucleosidzwischenprodukte mit Natriumhydrid oder DBU und Kohlenstoffdiselenid, um die Übergangsnucleoside 3'-O-(Z-substituierte) Selenophosphonsäuren zu bilden;
(c) Reagieren der Übergangsnucleoside mit einem Alkylierungsmittel der Formel R₈W, wobei R₈ CH₂C₆H₄R₇ ist, wobei R₇ H, Cl oder NO₂ ist und W ist C, Br oder I, um die alkylierten Nucleosidzwischenprodukte der Formeln (11a) und (11b) zu bilden 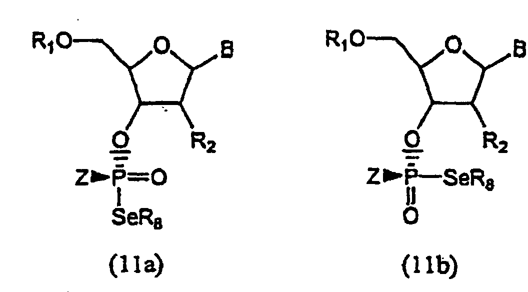 und
(d) Reagieren der alkylierten Nucleosidzwischenprodukte mit einem Nucleosid der Formel (5) 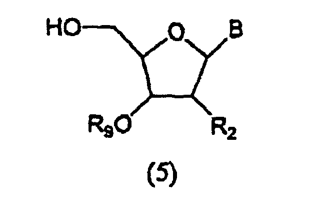 in Anwesenheit eines Aktivators und eines Lithiumsalzes in einem aprotischen organischen Lösungsmittel, um die diastereomere Mischung von P-chiralen Dinucleosiden der Formeln (6a) und (6b) zu bilden.

5. Verfahren zur Synthese eines chiral reinen Dinucleosids der Formel (6a) 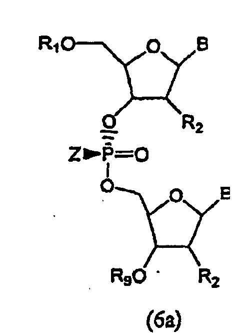 worin
R₁ eine Schutzgruppe ist;
R₂ ist H, OH oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (8a) 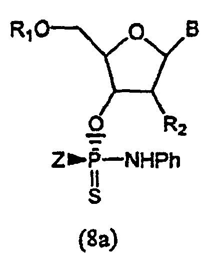 mit Natriumhydrid oder 1, 8-Diazabicyclo[5.4-0]undec-7-en und Kohlenstoffdioxid, um das Übergangsnucleosid 3'-O-(Z-substituierte) Thiophosphonsäure zu bilden;
(b) Reagieren des Übergangsnucleosids mit einem Alkylierungsmittel der Formel R₈W, wobei R₈ CH₂C₆H₄R₇ ist, wobei R₇ H, Cl oder NO₂ ist und W ist Cl, Br oder I, um alkyliertes Nucleosidzwischenprodukt der Formel (7a) zu bilden 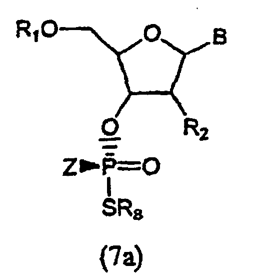
(c) Reagieren des alkylierten Nuceleosidzwischenprodukts mit einem Nucleosid der Formel (5) 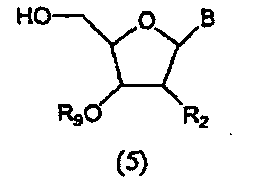 in Anwesenheit eines Aktivators und eines Lithiumsalzes in einem aprotischen organischen Lösungsmittel, um das chiral reine Dinucleosid der Formel (6a) zu bilden.

6. Verfahren zur Synthese eines chiral reinen Dinucleosids der Formel (6a) 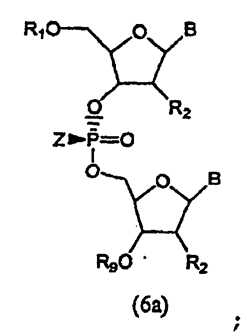 worin
R₁ eine Schutzgruppe ist;
R₂ ist H, OH oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (10a) 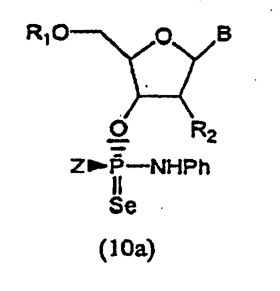 mit Natriumhydrid oder 1,8-Diazabicyclo[5.4.0]undec-7-en und Kohlendioxid, um das Übergangsnucleosid 3'-O-(Z-substituierte)Selenophosphonsäure zu bilden;
(b) Reagieren des Übergangsnucleosids mit einem Alkylierungsmittel der Formel R₈W, worin R₈ CH₂C₆H₄R₇ ist, wobei R₇ H, Cl oder NO₂ ist und W ist Cl, Br oder I, um alkyliertes Nucleosidzwischenprodukts der Formel (11a) zu erhalten 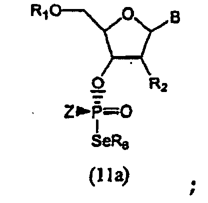 und
(c) Reagieren des alkylierten Nucleosidzwischenprodukts mit einem Nucleosid der Formel (5) 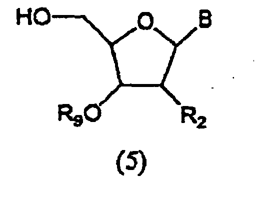 in Anwesenheit eines Aktivators und eines Lithiumsalzes in einem aprotischen organischen Lösungsmittels, um das chiral reine Dinucleosid der Formel (6a) zu bilden.

7. Verfahren zur Synthese eines chiral reinen Dinucleosids der Formel (6a) 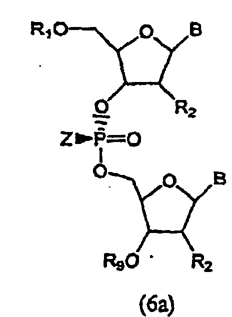 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (8b) 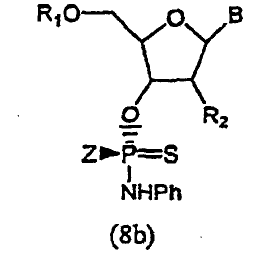 mit einem Kaliumperoxymonosulfat oder Wasserstoffperoxid, um ein Nucleosidzwischenprodukt der Formel (9b) zu bilden 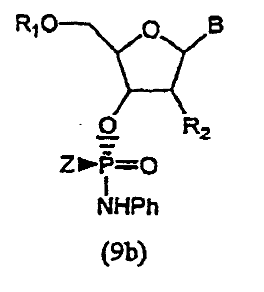
(b) Reagieren des Nucleosidzwischenprodukts mit Natriumhydrid oder DBU und Kohlenstoffdisulfid, um das Übergangsnucleosid 3'-O-(Z-substituierte) Thiophosphonsäure zu bilden;
(c) Reagieren des Übergangsnucleosids mit einem Alkylierungsmittel der Formel R₈W, wobei R₈ CH₂C₆H₄R₇ ist, wobei R₇ H, Cl oder NO₂ ist und W ist Cl, Br oder I, um ein alkyliertes Nucleosidzwischenprodukt der Formel (7a) zu bilden 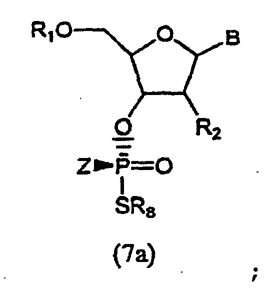 und
(d) Reagieren des alkylierten Nucleosidzwischenprodukts mit einem Nucleosid der Formel (5) 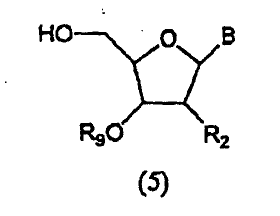 in Anwesenheit eines Aktivators und eines Lithiumsalzes in einem aprotischen organischen Lösungsmittels, um das chiral reine Dinucleosid der Formel (6a) zu bilden.

8. Verfahren zur Synthese eines chiral reinen Dinucleosids der Formel (6a) 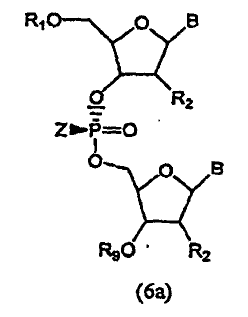 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren des Nucleosids der Formel (10b) 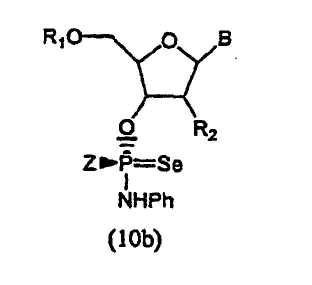 mit Kaliumperoxymonosulfat oder Wasserstoffperoxid, um das Nucleosidzwischenprodukt der Formel (9b) zu bilden 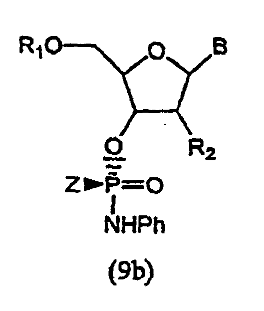
(b) Reagieren des Nucleosidzwischenprodukts mit Natriumhydrid oder 1,8-Diazabicyclo[5.4.0]undec-7-en und Kohlenstoffdiselenid, um das Übergangsnucleosid 3'-O-(Z-substituierte) Selenophosphonsäure zu bilden;
(c) Reagieren des Übergangsnucleosids mit einem Alkylierungsmittel der Formel R₈W, wobei R₈ CH₂C₆H₄R₇ ist, wobei R₇ H, Cl oder NO₂ ist und W ist C, Br oder I, um das alkylierte Nucleosidzwischenprodukt der Formel (11a) zu bilden 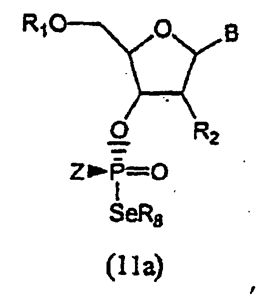 und
(d) Reagieren des alkylierten Nucleosidzwischenprodukts mit einem Nucleosid der Formel (5) 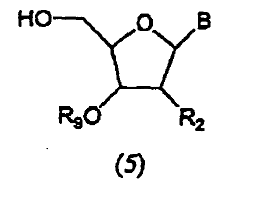 in Anwesenheit eines Aktivators und eines Lithiumsalzes in einem aprotischen organischen Lösungsmittels, um das chiral reine Dinucleosid der Formel (6a) zu bilden.

9. Verfahren zur Synthese eines chiral reinen Dinucleosids der Formel (6b) 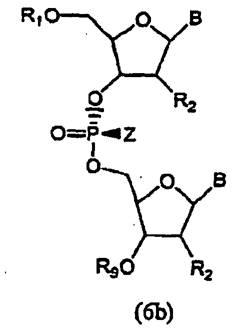 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (8b) 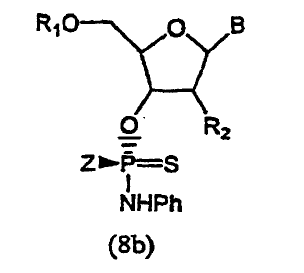 mit Natriumhydrid oder 1, 8-Diazabicyclo[5.4.0]undec-7-en und Kohlenstoffdioxid, um das Übergangsnucleosid 3'-O-(Z-substituierte)Thiophosphonsäure zu bilden;
(b) Reagieren des Übergangsnucleosids mit einem Alkylierungsmittel der Formel R₈W, wobei R₈ CH₂C₆H₄R₇ ist, wobei R₇ H, Cl oder NO₂ ist und W ist Cl, Br oder I, um alkyliertes Nucleosidzwischenprodukt der Formel (7b) zu bilden 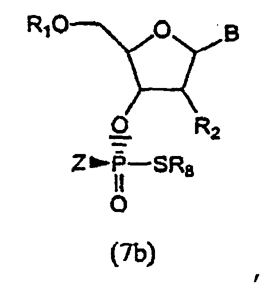 und
(c) Reagieren des alkylierten Nuceleosidzwischenprodukts mit einem Nucleosid der Formel (5) 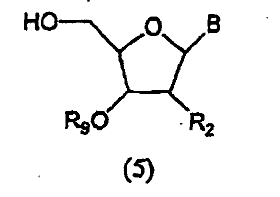 in Anwesenheit eines Aktivators und eines Lithiumsalzes in einem aprotischen organischen Lösungsmittel, um das chiral reine Dinucleosid der Formel (6b) zu bilden.

10. Verfahren zur Synthese eines chiral reinen Dinucleosids der Formel (6b) 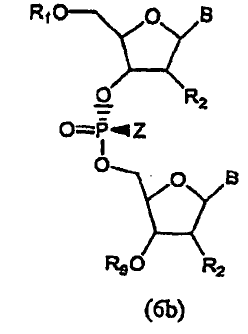 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren des Nucleosids der Formel (10b) 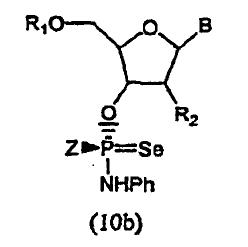 mit Natriumhydrid oder 1, 8-Diazabicyclo[5.4.0]undec-7-en und Kohlendioxid, um das Übergangsnucleosid 3'-O-(Z-substituierte)Selenophosphonsäure zu bilden;
(b) Reagieren des Übergangsnucleosids mit einem Alkylierungsmittel der Formel R₈W, worin R₈ CH₂C₆H₄R₇ ist, wobei R₇ H, Cl oder NO₂ ist und W Cl, Br oder I, um alkyliertes Nucleosidzwischenprodukt der Formel (11b) zu erhalten 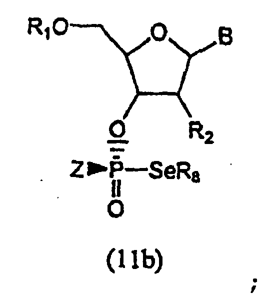 und
(c) Reagieren des alkylierten Nucleosidzwischenprodukts mit einem Nucleosid der Formel (5) 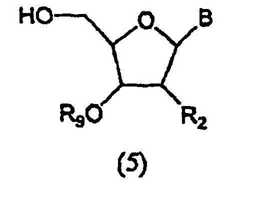 in Anwesenheit eines Aktivators und eines Lithiumsalzes in einem aprotischen organischen Lösungsmittel, um das chiral reine Dinucleosid der Formel (6b) zu bilden.

11. Verfahren zur Synthese eines chiral reinen Dinucleosids der Formel (6b) 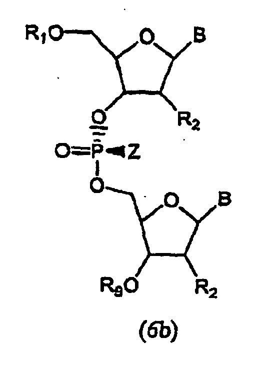 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren des Nucleosids der Formel(8a) 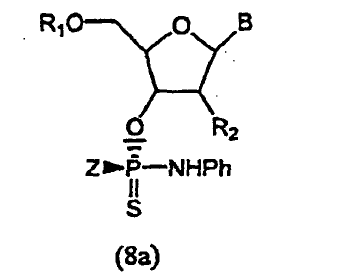 mit einem Kaliumperoxymonosulfat oder Wasserstoffperoxid, um ein Nucleosidzwischenprodukt der Formel (9a) zu bilden 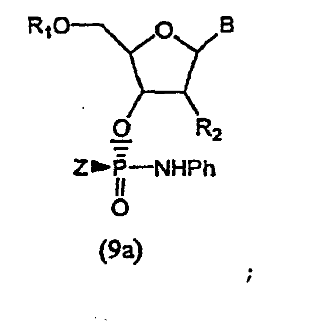
(b) Reagieren des Nucleosidzwischenprodukts mit Natriumhydrid oder DBU und Kohlenstoffdisulfid, um Übergangsnucleosid 3'-O-(Z-substituierte) Thiophosphonsäure zu bilden;
(c) Reagieren des Übergangsnucleosids mit einem Alkylierungsmittel der Formel R₈W, wobei R₈ CH₂C₆H₄R₇ ist, wobei R₇ H, Cl oder NO₂ ist und W ist Cl, Br oder I, um ein alkyliertes Nucleosidzwischenprodukt der Formel (7b) zu bilden 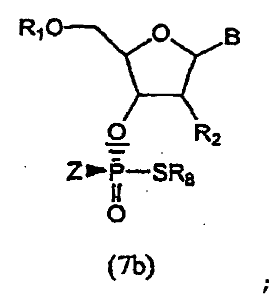 und
(d) Reagieren des alkylierten Nucleosidzwischenprodukts mit einem Nucleosid der Formel (5) 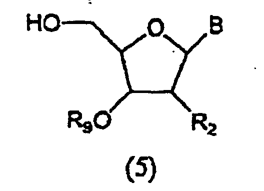 in Anwesenheit eines Aktivators und eines Lithiumsalzes in einem aprotischen organischen Lösungsmittel, um das chiral reine Dinucleosid der Formel (6b) zu bilden.

12. Verfahren zur Synthese eines chiral reinen Dinucleosids der Formel (6b) 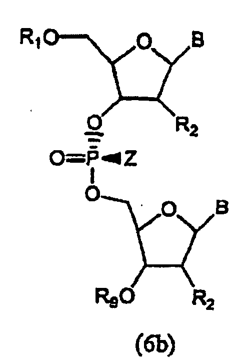 worin
R₁ eine Schutzgruppe ist;
R₂ ist H oder ein Alkoxyanteil von 1 bis 10 Kohlenstoffatomen;
R₉ ist eine Acylschutzgruppe, eine Kopplungsgruppe oder eine Silylschutzgruppe;
Z ist ein Alkylanteil, ein Aralkylanteil, ein Haloalkylanteil, ein Alkenylanteil, ein Alkinylanteil, ein Alkarylanteil, ein Arylanteil von 1 bis 20 Kohlenstoffatomen, ein Aminomethyl oder ein Aminoethyl;
B ist eine N-geschützte Nucleosidbase;
umfassend die Schritte:
(a) Reagieren der Nucleoside der Formel (10a) 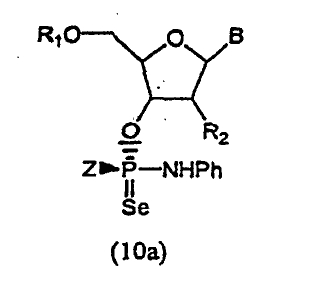 mit Kaliumperoxymonosulfat oder Wasserstoffperoxid, um das Nucleosidzwischenprodukt der Formel (9a) zu bilden 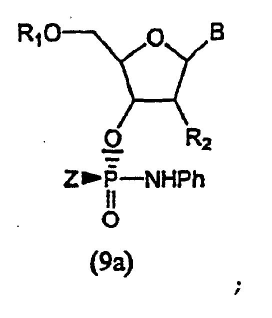
(b) Reagieren des Nucleosidzwischenprodukts mit Natriumhydrid oder 1,8-Diazabicyclo[5.4.0]undec-7-en und Kohlenstoffdiselenid, um ein Übergangsnucleosid 3'-O-(Z-substituierte) Selenophosphonsäure zu bilden;
(c) Reagieren des Übergangsnucleosids mit einem Alkylierungsmittel der Formel R₈W, wobei R₈ CH₂C₆H₄R₇ ist, wobei R₇ H, Cl oder NO₂ ist und W ist C, Br oder I, um das alkylierte Nucleosidzwischenprodukt der Formel (11b) zu bilden 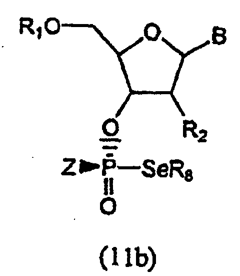 und
(d) Reagieren des alkylierten Nucleosidzwischenprodukts mit einem Nucleosid der Formel (5) 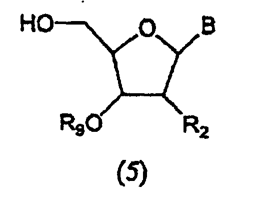 in Anwesenheit eines Aktivators und eines Lithiumsalzes in einem aprotischen organischen Lösungsmittel, um das chiral reine Nucleosid der Formel (6b) zu bilden.

13. Verfahren gemäß einem der Ansprüche 3 bis 12, wobei das aprotische Lösungsmittel Acetonitril ist.

14. Verfahren gemäß einem der Ansprüche 3 bis 12, wobei der Aktivator 1,8-Diazabicyclo[5.4.0] undec-7-en ist.

## Revendications

1. Procédé pour la synthèse d'un mélange diastéréoisomère de dinucléosides P-chiraux de formules (6a) et (6b) 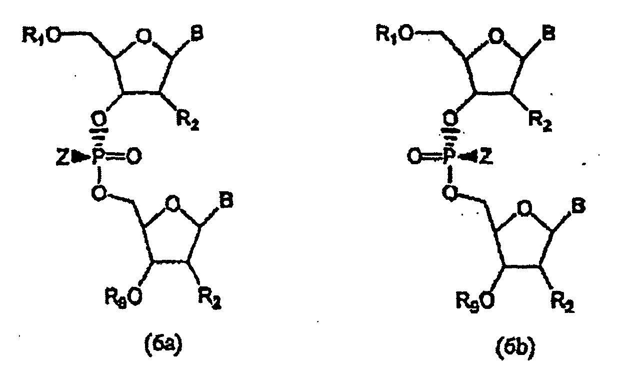 dans lesquelles
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a)réaction des nucléosides de formules (8a) et (8b) 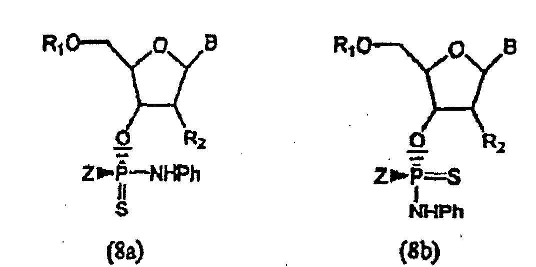 avec l'hydrure de sodium ou le 1,8-diazabicyclo[5.4.0]undéc-7-ène et du dioxyde de carbone pour former des nucléosides acides 3'-O-(Z-substitués)phosphonothioïques transitoires ;
(b) réaction des nucléosides transitoires avec un agent alkylant de formule R₈W, dans laquelle R₈ est CH₂C₆H₄R₇, dans laquelle R₇ est H, Cl, ou NO₂, et W est Cl, Br, ou I, pour former des intermédiaires nucléosidiques alkylés de formules (7a) et (7b) 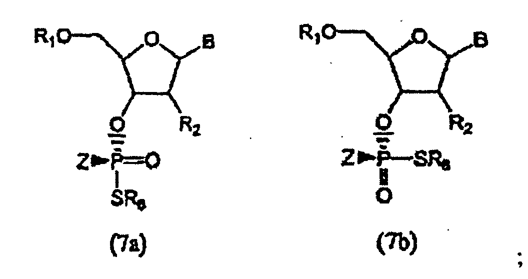 et
(c) réaction des intermédiaires nucléosidiques alkylés avec un nucléoside de formule (5) 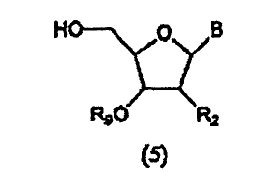 en présence d'un activateur et d'un sel de lithium dans un solvant organique aprotique pour former le mélange diastéréoisomère de dinucléosides P-chiraux de formules (6a) et (6b).

2. Procédé pour la synthèse d'un mélange diastéréoisomère de dinucléosides P-chiraux de formules (6a) et (6b) 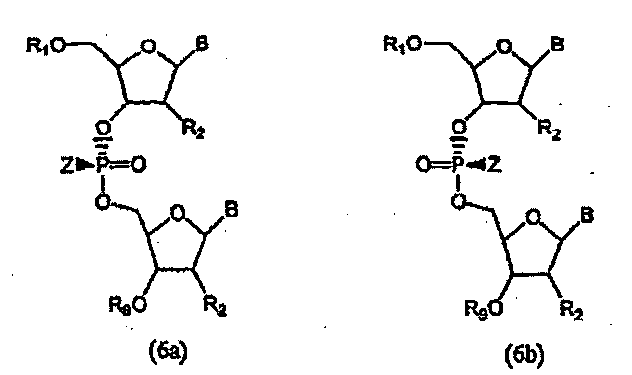 dans lesquelles
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidiqué N-protégée ;
comprenant les étapes de :
(a) réaction des nucléosides des formules (10a) et (10b) 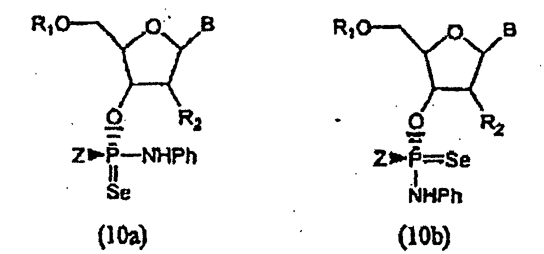 avec l'hydrure de sodium ou le 1,8-diazabicyclo[5.4.0]undéc-7-ène et du dioxyde de carbone pour former des nucléosides acides 3'-O-(Z-substitués)phosphonosélénoïques transitoires ;
(b) réaction des nucléosides transitoires avec un agent alkylant de formule R₈W, dans laquelle R₈ est CH₂C₆H₄R₇, dans laquelle R₇ est H, Cl, ou NO₂, et W est Cl, Br, ou I, pour former des intermédiaires nucléosidiques alkylés de formules (11a) et (11b) 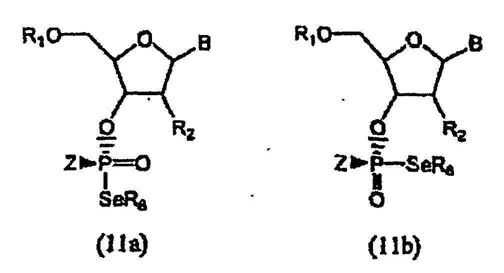 ; et
(c ) réaction des intermédiaires nucléosidiques alkylés avec un nucléoside de formule (5) 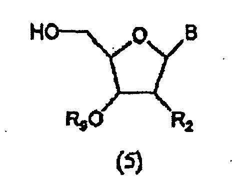 en présence d'un activateur et d'un sel de lithium dans un solvant organique aprotique pour former le mélange diastéréoisomère de dinucléosides P-chiraux de formules (6a) et (6b).

3. Procédé pour la synthèse d'un mélange diastéréoisomère de dinucléosides P-chiraux de formules (6a) et (6b) 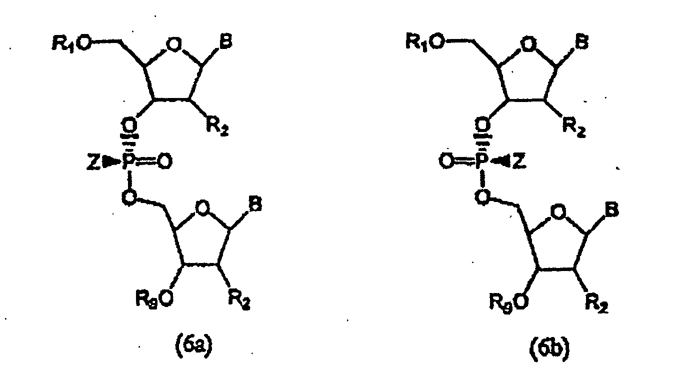 dans lesquelles
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction des nucléosides des formules (8a) et (8b) 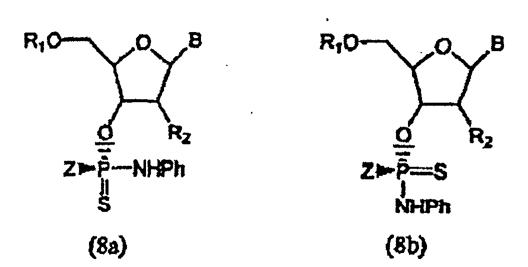 avec le peroxymonosulfate de potassium ou le peroxyde d'hydrogène pour former des intermédiaires nucléosidiques des formules (9a) et (9b) 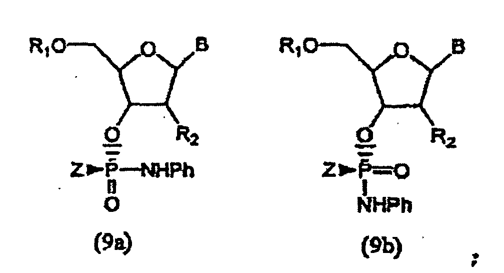
(b) réaction des intermédiaires nucléosidiques avec l'hydrure de sodium ou le 1,8-diazabicyclo[5.4.0]undéc-7-ène et du disulfure de carbone pour former des nucléosides acides 3'-O-(Z-substitués)phosphonothioïques transitoires ;
(c) réaction des nucléosides transitoires avec un agent alkylant de formule R₈W, dans laquelle R₈ est CH₂C₆H₄R₇, dans laquelle R₇ est H, Cl, ou NO₂, et W est Cl, Br, ou I, pour former des intermédiaires nucléosidiques alkylés de formules (7a) et (7b) 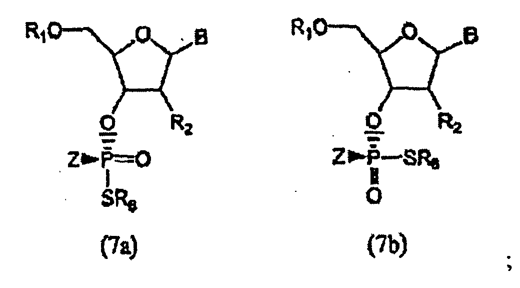 et
(d) réaction des intermédiaires nucléosidiques alkylés avec un nucléoside de formule (5) 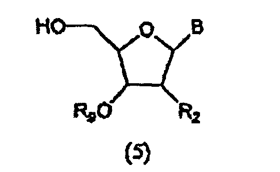 en présence d'un activateur et d'un sel de lithium dans un solvant organique aprotique pour former le mélange diastéréoisomère de dinucléosides P-chiraux de formules (6a) et (6b).

4. Procédé pour la synthèse d'un mélange diastéréoisomère de dinucléosides P-chiraux de formules (6a) et (6b) 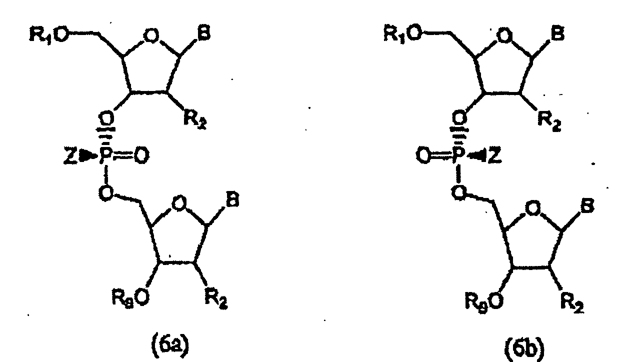 dans lesquelles
R₁ est un groupe de protection ;
R₂ est H ou OH, ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction des nucléosides des formules (10a) et (10b) 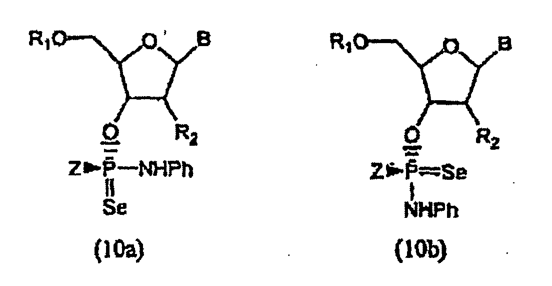 avec le peroxymonosulfate de potassium ou le peroxyde d'hydrogène pour former des intermédiaires nucléosidiques de formules (9a) et (9b) 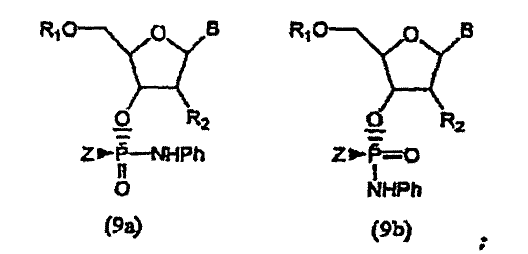
(b) réaction des intermédiaires nucléosidiques avec l'hydrure de sodium ou le DBU et le diséléniure de carbone pour former des nucléosides acides 3'-O-(Z-substitués)phosphonosélénoïques transitoires ;
(c) réaction des nucléosides transitoires avec un agent alkylant de formule R₈W, dans laquelle R₈ est CH₂C₆H₄R₇, dans laquelle R₇ est H, Cl, ou NO₂, et W est Cl, Br, ou I, pour former des intermédiaires nucléosidiques alkylés de formules (11a) et (11b) 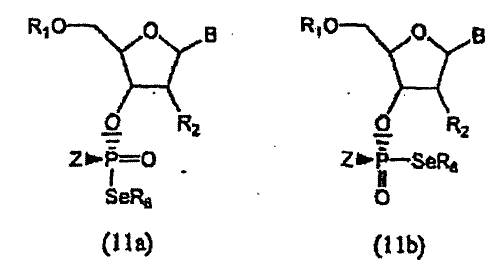 ; et
(d) réaction des intermédiaires nucléosidiques alkylés avec un nucléoside de formule (5) 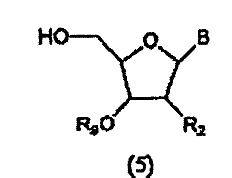 en présence d'un activateur et d'un sel de lithium dans un solvant organique aprotique pour former le mélange diastéréoisomère de dinucléosides P-chiraux de formules (6a) et (6b).

5. Procédé de synthèse d'un dinucléoside chiralement pur de formule (6a) 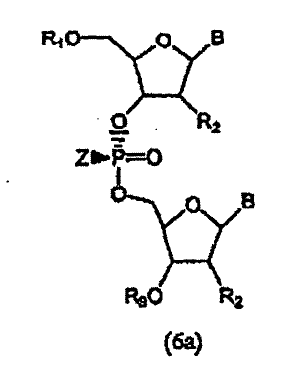 dans laquelle
R₁ est un groupe de protection ;
R₂ est H ou OH, ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a)réaction d'un nucléoside de formule (8a) 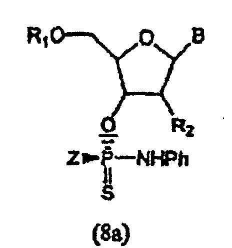 avec l'hydrure de sodium ou le 1,8-diazabicyclo[5.4.0]undéc-7-ène et du dioxyde de carbone pour former un nucléoside acide 3'-O-(Z-substitués)phosphonothioïque transitoire ;
(b) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (7a) ; 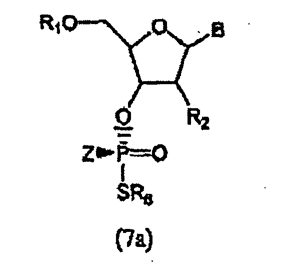 et
(c) réaction de l'intermédiaire nucléosidique alkylé avec un nucléoside de formule (5) 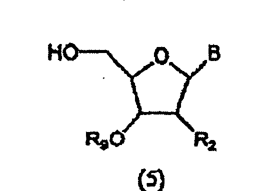 en présence d'un activateur et d'un sel de lithium dans un solvant organique aprotique pour former le dinucléoside chiralement pur de formule (6a).

6. Procédé de synthèse d'un dinucléoside chiralement pur de formule (6a) 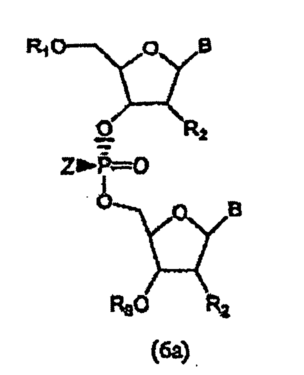 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (10a) 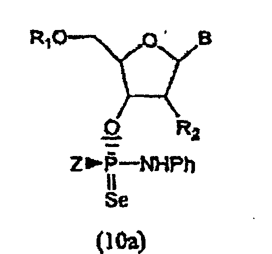 avec l'hydrure de sodium ou le 1,8-diazabicyclo[5.4.0]undéc-7-ène et du dioxyde de carbone pour former un nucléoside acide 3'-O-(Z-substitués)phosphonosélénoïque transitoire ;
(b) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (11a) ; 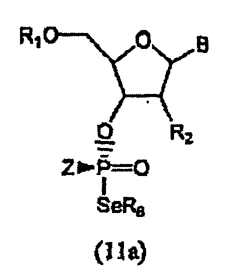 et
(c) réaction de l'intermédiaire nucléosidique alkylé avec un nucléoside de formule (5) 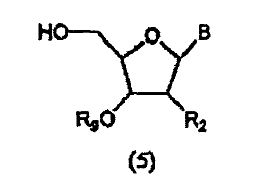 en présence d'un activateur et d'un sel de lithium dans un solvant organique aprotique pour former le dinucléoside chiralement pur de formule (6a).

7. Procédé de synthèse d'un dinucléoside chiralement pur de formule (6a) 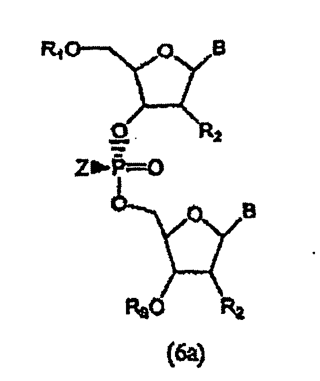 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (8b) 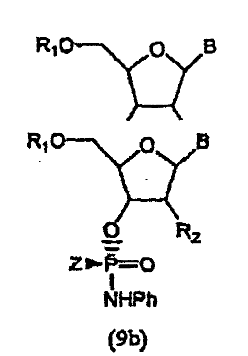 avec le peroxymonosulfate de potassium ou le peroxyde d'hydrogène pour former un intermédiaire nucléosidique de formule (9b)
(b) réaction de l'intermédiaire nucléosidique avec l'hydrure de sodium ou le DBU et le disulfure de carbone pour former un nucléoside acide 3'-O-(Z-substitué)phosphonothioïque transitoire ;
(c) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (7a) 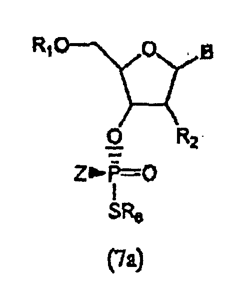 et
(d) réaction de l'intermédiaire nucléosidique alkylé avec un nucléoside de formule (5) 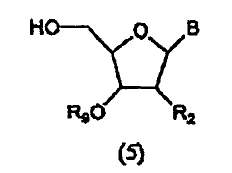 en présence d'un activateur et d'un sel de lithium dans un solvant organique aprotique pour former le dinucléoside chiralement pur de formule (6a).

8. Procédé de synthèse d'un dinucléoside chiralement pur de formule (6a) 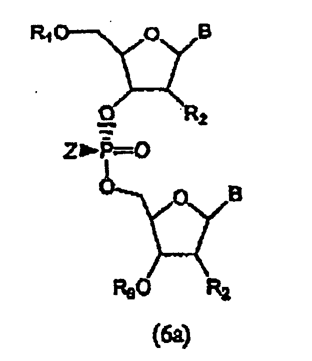 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (10b) 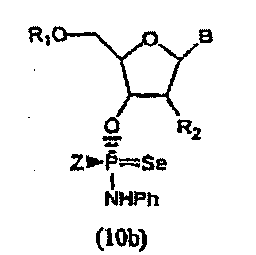 avec le peroxymonosulfate de potassium ou le peroxyde d'hydrogène pour former un intermédiaire nucléosidique de formule (9b) 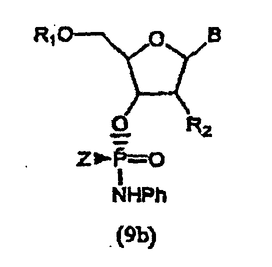
(b) réaction de l'intermédiaire nucléosidique avec l'hydrure de sodium ou le 1,8-diazabicyclo[5.4.0]undéc-7-ène et le diséléniure de carbone pour former un nucléoside acide 3'-O-(Z-substitué)phosphonosélénoïque transitoire ;
(c) réaction des nucléosides transitoires avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (11a) 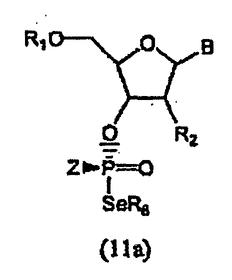 et
(d) réaction de l'intermédiaire nucléosidique alkylé avec un nucléoside de formule (5) 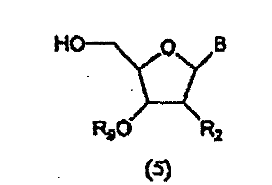 en présence d'un activateur et d'un sel de lithium dans un solvant organique aprotique pour former le dinucléoside chiralement pur de formule (6a).

9. Procédé de synthèse d'un dinucléoside chiralement pur de formule (6b) 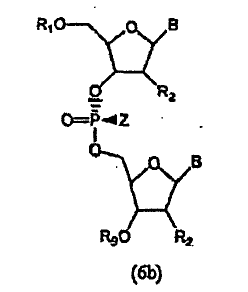 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (8b) 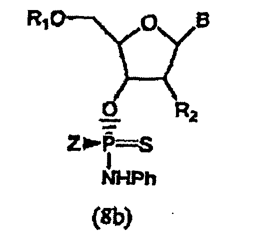 avec l'hydrure de sodium ou le 1,8-diazabicyclo[5.4.0]undéc-7-ène et du dioxyde de carbone pour former un nucléoside acide 3'-O-(Z-substitués)phosphonothioïques transitoire ;
(b) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (7b) ; 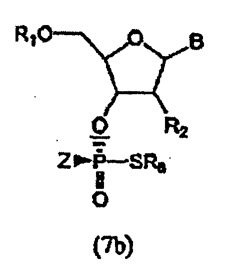 et
(c) réaction de l'intermédiaire nucléosidique alkylé avec un nucléoside de formule (5) 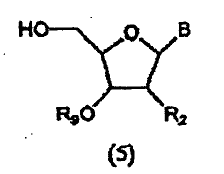 en présence d'un activateur et d'un sel de lithium dans un solvant organique aprotique pour former le dinucléoside chiralement pur de formule (6a).

10. Procédé de synthèse d'un dinucléoside chiralement pur de formule (6b) 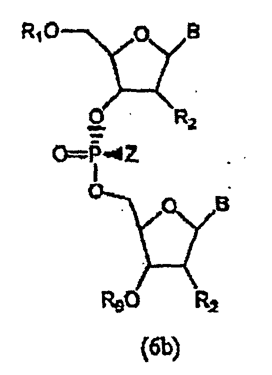 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (10b) 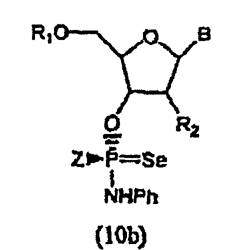 avec l'hydrure de sodium ou le 1,8-diazabicyclo[5.4.0]undéc-7-ène et du dioxyde de carbone pour former un nucléoside acide 3'-O-(Z-substitué)phosphonosélénoïque transitoire ;
(b) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou 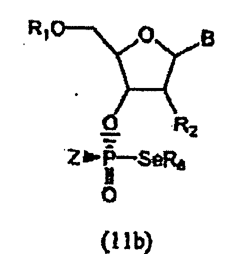 I, pour former un intermédiaire nucléosidique alkylé de formule (11b) ;
et
(c) réaction de l'intermédiaire nucléosidique alkylé avec un nucléoside de formule (5) 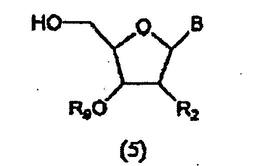 en présence d'un activateur et d'un sel de lithium dans un solvant organique aprotique pour former le dinucléoside chiralement pur de formule (6a).

11. Procédé de synthèse d'un dinucléoside chiralement pur de formule (6b) 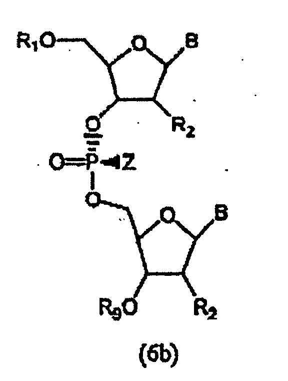 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (8a) 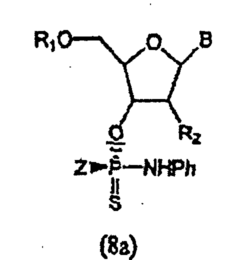 avec le peroxymonosulfate de potassium ou le peroxyde d'hydrogène pour former un intermédiaire nucléosidique de formule (9a) 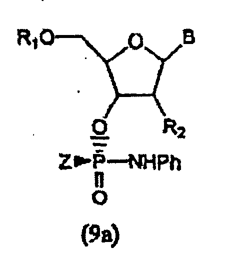
(b) réaction de l'intermédiaire nucléosidique avec l'hydrure de sodium ou le DBU et le disulfure de carbone pour former un nucléoside acide 3'-O-(Z-substitué)phosphonothioïque transitoire ;
(c)réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (7b) ; 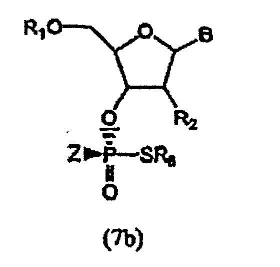 et
(d) réaction de l'intermédiaire nucléosidique alkylé avec un nucléoside de formule (5) 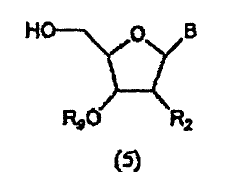 en présence d'un activateur et d'un sel de lithium dans un solvant organique aprotique pour former le dinucléoside chiralement pur de formule (6a).

12. Procédé de synthèse d'un dinucléoside chiralement pur de formule (6b) 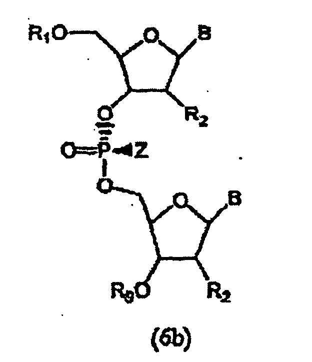 dans laquelle
R₁ est un groupe de protection ;
R₂ est H ou une fraction alcoxy de 1 à 10 carbones ;
R₉ est un groupe de protection acyle, un groupe de couplage, ou un groupe de protection silyle ;
Z est une fraction alkyle, une fraction aralkyle, une fraction halogénoalkyle, une fraction alcényle, une fraction alkynyle, une fraction alkaryle, une fraction aryle de 1 à 20 carbones, un aminométhyle, ou un aminoéthyle ;
B est une base nucléosidique N-protégée ;
comprenant les étapes de :
(a) réaction d'un nucléoside de formule (10a) 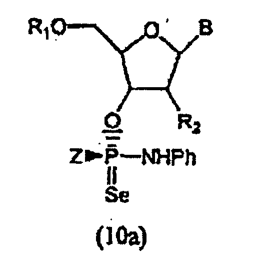 avec le peroxymonosulfate de potassium ou le peroxyde d'hydrogène pour former un intermédiaire nucléosidique de formule (9a) 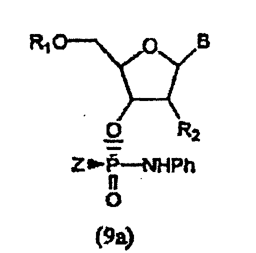
(b) réaction de l'intermédiaire nucléosidique avec l'hydrure de sodium ou le 1,8-diazabicyclo[5.4.0]undéc-7-ène et le diséléniure de carbone pour former un nucléoside acide 3'-O-(Z-substitué)phosphonosélénoïque transitoire ;
(c) réaction du nucléoside transitoire avec un agent alkylant de formule R₈W, dans laquelle R₈ est CH₂C₆H₄R₇, dans laquelle R₇ est H, Cl, ou NO₂, et W est Cl, Br, ou I, pour former un intermédiaire nucléosidique alkylé de formule (11b) ; 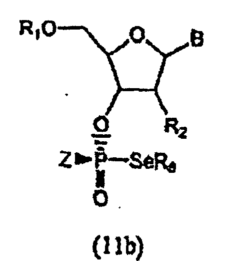 et
(d) réaction de l'intermédiaire nucléosidique alkylé avec un nucléoside de formule (5) 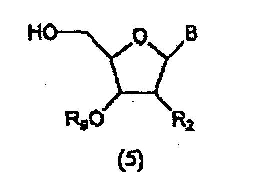 en présence d'un activateur et d'un sel de lithium dans un solvant organique aprotique pour former le dinucléoside chiralement pur de formule (6a).

13. Procédé selon l'une quelconque des revendications 3 à 12, dans lequel le solvant aprotique est l'acétonitrile.

14. Procédé selon l'une quelconque des revendications 3 à 12, dans lequel l'activateur est le 1,8-diazabicyclo[5.4.0]undéc-7-ène.
